(19) **Europäisches Patentamt / European Patent Office / Office européen des brevets**

(11) **EP 4 190 913 A1**

(12) **EUROPEAN PATENT APPLICATION**

(43) Date of publication:
**07.06.2023 Bulletin 2023/23**

(21) Application number: **21383088.8**

(22) Date of filing: **02.12.2021**

(51) International Patent Classification (IPC):
**C12Q 1/6816** (2018.01)      **C12Q 1/70** (2006.01)

(52) Cooperative Patent Classification (CPC):
(C-Sets available)
**C12Q 1/702; C12Q 1/6816**                    (Cont.)

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **Fundación Imdea Nanociencia**
**28049 Madrid (ES)**

(72) Inventors:
• **RODRÍGUEZ DÍAZ, Ciro**
  **E-28049 Madrid (ES)**
• **LAFUENTE GÓMEZ, Nuria**
  **E-28049 Madrid (ES)**
• **CASTANHEIRA COUTINHO, Catarina**
  **E-28049 Madrid (ES)**
• **CASTELLANOS MOLINA, Milagros**
  **E-28049 Madrid (ES)**
• **SOMOZA CALATRAVA, Álvaro**
  **E-28049 Madrid (ES)**

(74) Representative: **ABG Intellectual Property Law, S.L.**
**Avenida de Burgos, 16D**
**Edificio Euromor**
**28036 Madrid (ES)**

Remarks:
The complete document including Reference Table(s) and the Sequence Listing(s) can be downloaded from the EPO website

(54) **FUNCTIONALIZED METAL NANOPARTICLES AND USES THEREOF FOR DETECTING SARS COV 2 GENETIC MATERIAL**

(57)    The present invention relates to a metal nanoparticle bound to an oligonucleotide, wherein said oligonucleotide comprises a first nucleotide region, a second nucleotide region and a third nucleotide region, in that order, wherein said first and third regions are at least partially complementary such that they can give rise to a hairpin-type structure by means of hybridization, wherein said second polynucleotide region is at least partially complementary to a region of the SARS-CoV-2 genome and wherein said oligonucleotide is modified with a hydrophobic molecule at the end opposite the end bound to the metal nanoparticle.

EP 4 190 913 A1

(52) Cooperative Patent Classification (CPC): (Cont.)

C-Sets
**C12Q 1/6816, C12Q 2525/301, C12Q 2563/137,
C12Q 2563/155, C12Q 2565/113**

**Description**

**FIELD OF THE INVENTION**

The present invention relates to metal nanoparticles functionalized with an oligonucleotide which is partially complementary to the specific regions of the genetic material of SARS-CoV-2, to a method for the preparation thereof, to methods for detecting the presence of SARS-CoV-2 genetic material in a sample using said nanoparticles, as well as to kits comprising the reagents necessary for obtaining said nanoparticles.

**BACKGROUND OF THE INVENTION**

Severe acute respiratory syndrome coronavirus 2 (SARS-CoV-2) was identified in December 2019 in Wuhan (China) as the cause of the illness designated COVID-19, and it has rapidly spread all around the globe. As a matter of fact, the ongoing SARS-CoV-2 pandemic is far from being solved, and its surveillance should be maintained because a resurgence in contagion could be possible as late as 2024. In this scenario, the relatively low seroprevalence, especially in low and median income countries, is insufficient to provide herd immunity. Universal vaccination still will take a long time and faces the challenge of virus mutations, which can reduce or even prevent an adequate immunization. Therefore, fast, reliable, and efficient diagnostic methods for the rapid identification and isolation of infected patients, as well as contact tracing, are foremost priorities to prevent the virus from spreading.

The gold-standard method for SARS-CoV-2 detection is the reverse transcription quantitative polymerase chain reaction (RT-qPCR) test performed using nasopharyngeal swabs to target a variety of specific RNA viral sequences. There are different proposed workflows, being the most prevalently used in Europe a protocol in which the first step consists of detecting all SARS-related viruses by targeting regions of the E gene, common for all Sarbecovirus subgenus members. If the test is positive, then one proceeds to detecting the region of the ORF1ab gene which encodes for the R protein, which is specific to SARS-CoV-2. The continuous emergence of single-nucleotide polymorphisms and deletions that might adversely affect RT-qPCRs justifies that at least two genomic regions should be targeted to avoid false negatives in the molecular diagnosis of SARS-CoV-2.

RT-qPCR is widely used for its high specificity and sensitivity. However, this technique presents some disadvantages, such as the need for qualified personnel, sophisticated equipment, and expensive qPCR reagent kits, limiting its application due to the current huge demand. For these reasons, new fast, low-cost and reliable point-of-care devices are needed for the detection of SARS-CoV-2.

The use of gold nanoparticles has many applications in biology and medicine as mechanisms useful for transporting substances of interest, for example drugs. The use of metal nanoparticles as sensors stands out among other applications. In most cases, the use of gold nanoparticles as sensors is based on the change in color occurring in the solution in which the nanoparticles are found because when gold nanoparticles are in a colloidal dispersion, the color of the solution is reddish, whereas when said nanoparticles aggregate, the color of the solution becomes bluish.

Despite their wide use, metal nanoparticles are known to interact with several molecules and compounds, or even the oligonucleotides functionalized to said nanoparticles, which are close to their surface, affecting the stability of the nanoparticles in solution and therefore, the distance between the fluorophore and the surface of the nanoparticle is critical. In this sense, it has been described that the fluorescence of a fluorophore is deactivated when the distance to the metal is too short. For this reason the sensitivity of the methods using these functionalized gold nanoparticles does not allow distinguishing nucleotide alterations in the sequence of the nucleic acid of interest. Furthermore, the usual preparation of such nanoparticles requires synthesizing oligonucleotides substituted with thiol by means of using commercial solid supports containing a protected thiol group. After synthesis and purification of the oligonucleotide, the protected thiol group has to be deprotected using an excess of a reagent such as DTT or phosphine derivatives. The use of said reducing agents can alter oligonucleotide stability, this process being critical particularly when the oligonucleotide is RNA.

Therefore, there is a need in the state of the art to provide new fast, low-cost and reliable point-of-care devices for the detection of SARS-CoV-2 as well as to develop a method which allows detecting alterations in a sequence of a nucleic acid of interest by means of using nanoparticles.

**BRIEF DESCRIPTION OF THE INVENTION**

To address the drawbacks of the current methods of SARS-CoV-2 detection as well as the lack of sensitivity of nanoparticles based sensors the inventors developed a colorimetric test based on metal nanoparticles (NPs) for the detection of SARS-CoV-2 nucleic acids wherein the nanoparticles are functionalized with oligonucleotides modified with a hydrophobic molecule, wherein said oligonucleotides target a region of SARS-CoV-2 genome. When the target is present, the oligonucleotides unfold due to the hybridization between the target and the oligonucleotide, and the hydrophobic units become exposed to the aqueous media leading to a color change in the solution because of the destabilization, aggregation and precipitation of the NPs.

Therefore, a first aspect of the present invention relates to a metal nanoparticle bound to an oligonucleotide, wherein said oligonucleotide comprises a first nucleotide region, a second nucleotide region and a third nucleotide region, in that order, wherein said first and third regions are at least partially complementary such that they can give rise to a hairpin-type structure by means of hybridization, wherein said second polynucleotide region is at least partially complementary to a region of the SARS-CoV-2 genome and wherein said oligonucleotide is modified with a hydrophobic molecule at the end opposite the end bound to the metal nanoparticle.

Another aspect of the present invention relates to a process for obtaining a metal nanoparticle according to any of the preceding claims which comprises:

(i) contacting a metal nanoparticle with an oligonucleotide, wherein the oligonucleotide comprises a first, second and third region as defined in any of the preceding claims and wherein the oligonucleotide is modified with a group which is capable of reacting with the surface nanoparticle and

(ii) maintaining the nanoparticles and the oligonucleotides under suitable conditions for the formation of a bond between said oligonucleotide and the metal nanoparticle.

Yet another aspect of the present invention relates to a method for detecting the presence of a SARS-CoV-2 genetic material in a test sample which comprises:

(i) contacting a nanoparticle according to the invention with said sample wherein said contacting is carried out under conditions that allow hybridizing said region of the oligonucleotide of the nanoparticle with the nucleic acid of said sample; and

(ii) detecting aggregation of the nanoparticles, wherein an increase in aggregation with respect to a control sample indicates that the test sample contains SARS-CoV-2 genetic material.

One final aspect of the present invention relates to a kit comprising a metal nanoparticle according to the invention.

## BRIEF DESCRIPTION OF THE DRAWINGS

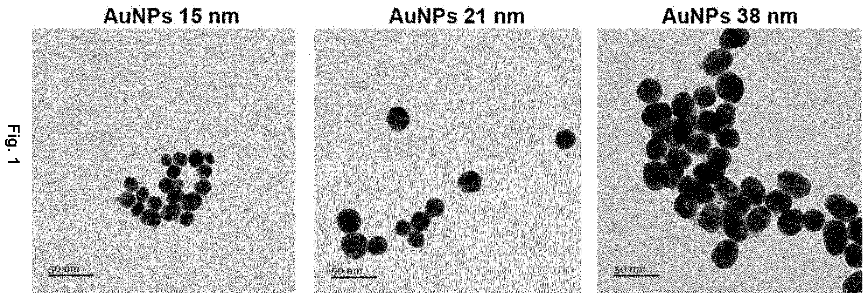

**Figure 1.** TEM images of AuNPs of different sizes in solution.

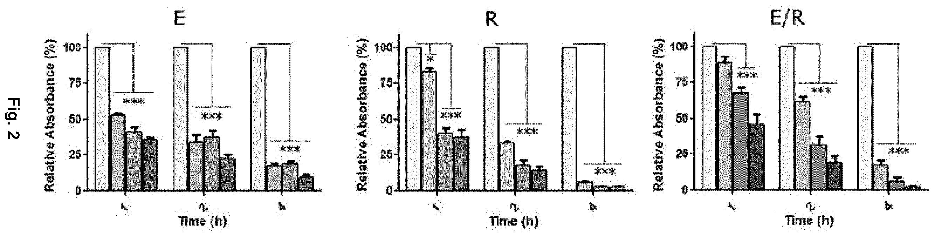

**Figure 2.** Optimization of the molecular beacons (MBs) for the detection of target DNA sequences mimicking the E and R SARS-CoV-2 specific gene regions. AuNPs of 21nm functionalized with the MBs E and R (alone or in combination) containing the 5nt stem were tested using different concentrations of target (0 for the negative control, 50, 100 and 250 nM from left to right in the graphs). Data are presented as mean $\pm$ SD (n=3) in terms of relative absorbance (%) vs negative control. Statistical analysis was performed using one-way ANOVA with Tukey's test (pairwise comparison). Significance codes: 0 '***' 0.001 '**' 0.01 '*' 0.05 '.' 0.1 '' 1.

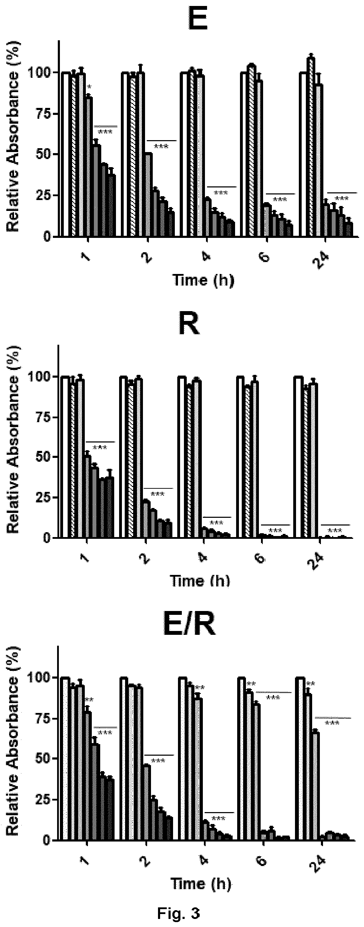

**Figure 3.** Optimization of the MBs for the detection of target RNA sequences mimicking the E and R SARS-CoV-2 specific gene regions. AuNPs of 21nm functionalized with the MBs E and R (alone or in combination) containing the 5nt stem were tested using different concentrations of target: E and R: 0 for the negative control (white), 1000nM for the alternative nonspecific target (striped), 1, 50, 100, 250 and 1000 nM from left to right in the graphs (greyscale); E/R: 0 for the negative control, 1, 5, 50, 100, 250 and 1000 nM from left to right in the graphs (greyscale). Data are presented as mean $\pm$ SD (n=3) in terms of relative absorbance (%) vs negative control. Statistical analysis was performed using one-way ANOVA Tukey's test (pairwise comparison). Signif. codes: 0 '***' 0.001 '**' 0.01 '*' 0.05 '.' 0.1 '' 1.

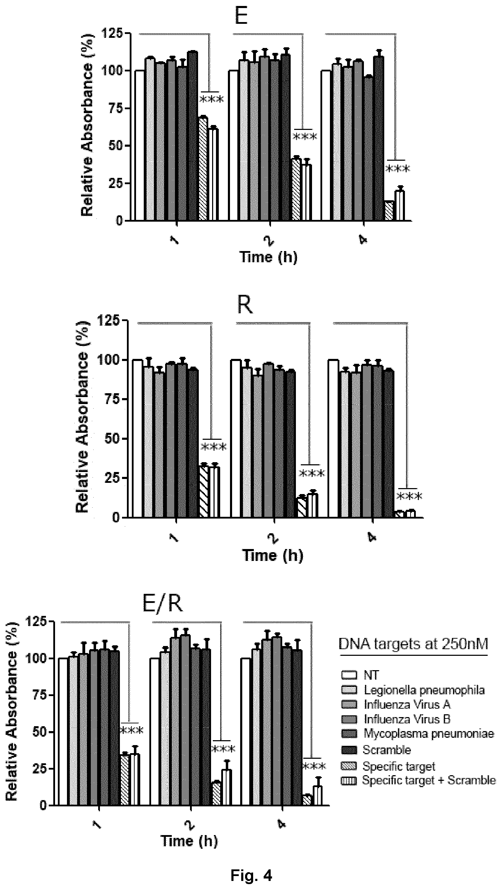

**Figure 4.** Gold Nanoparticles based sensors' selectivity for the detection of R and/or E RNA mimicking SARS-CoV-2 targets in the presence of other co-infection pathogens DNA sequences (Scramble). AuNPs of 23 nm were functionalized with MB E and/or R and tested using 250nM target (final concentration independently if they are alone or in combination). Data are presented as mean $\pm$ SD (n=3) in terms of relative absorbance (%) vs negative (NT) control. Statistical analysis was performed using one-way ANOVA with Tukey's test (pairwise comparison). Significance codes: 0 '***' 0.001 '**' 0.01 '*' 0.05 '.' 0.1 ' ' 1.

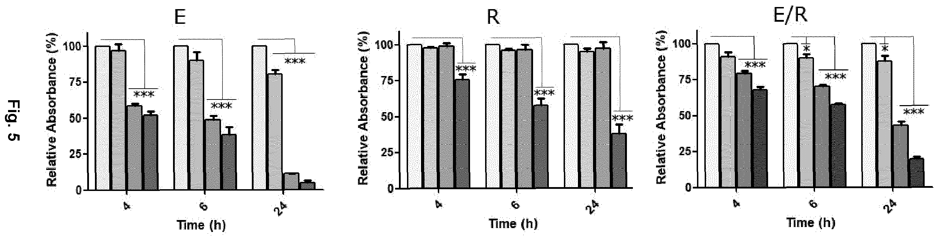

**Figure 5.** Gold Nanoparticles based sensors' detection limit. Titration experiments using targets R and/or E RNA mimicking SARS-CoV-2. AuNPs of 21nm functionalized with the MBs E and R (alone or in combination) containing the 5nt stem were tested using different concentrations of target (0 for the negative control, 1, 10 and 20 nM from left to right in the graphs). Data are presented as mean $\pm$ SD (n=3) in terms of relative absorbance (%) vs negative control. Statistical analysis was performed using one-way ANOVA with Tukey's test (pairwise comparison). Significance codes: 0 '***' 0.001 '**' 0.01 '*' 0.05 '.' 0.1 ' ' 1.

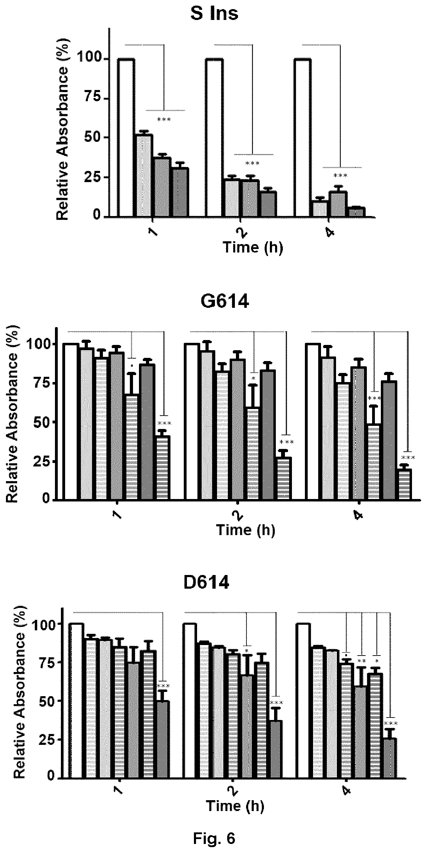

**Figure** 6. Gold Nanoparticles based sensors' functionality targeting the insertion region in S gene (S Ins) or the S mutation D614G. For the detection, DNA mimics of the different S regions were prepared and confronted to the complementary loops included in MB S Ins, G614 or D614 used to functionalize 23 nm AuNPs. Data are presented as mean ± SD (n=3) in terms of relative absorbance (%) vs negative control. The concentration of targets used (from left to right) were 0, 50 nM, 100 and 250 nM (for S Ins) or 0, 25, 50 and 100 nM (for G614 in stripped greyscale and D614 in grey). Statistical analysis was performed using one-way ANOVA Tukey's test (pairwise comparison). Significance codes: 0 '***' 0.001 '**' 0.01 '*' 0.05 '.' 0.1 '' 1.

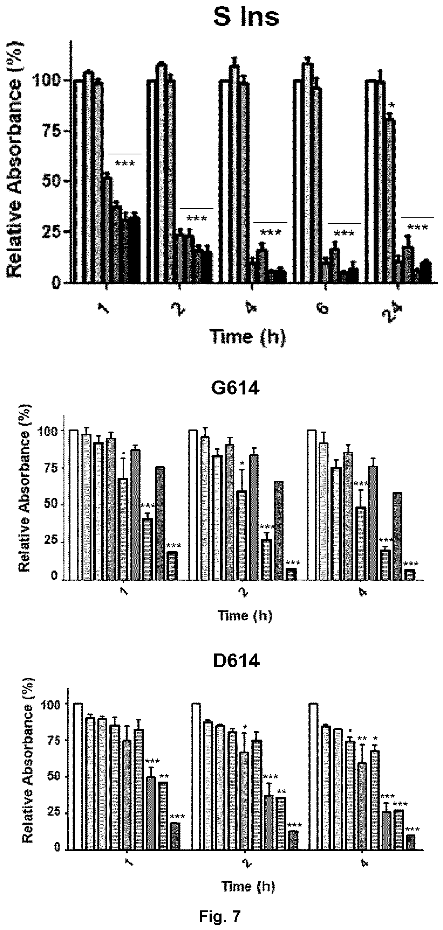

**Figure** 7. Gold Nanoparticles based sensors' functionality targeting the insertion region in S gene (S Ins) or the S mutation D614G. For the detection, DNA mimics of the different S regions were prepared and confronted to the complementary loops included in MB S Ins (grey), G614 (stripped greyscale) or D614 (grey) used for functionalize 23 nm AuNPs. Data are presented as mean ± SD (n=3) in terms of relative absorbance (%) vs negative control. The concentration of targets used (from left to right) was: 0, 0.25, 1, 50, 100, 250 and 1000 nM (for S Ins) or 0, 25, 50, 100 and 250 nM (for G614 and D614, both targets included in all experiments). Statistical analysis was performed using one-way ANOVA Tukey's test (pairwise comparison). Signif. codes: 0 '***' 0.001 '**' 0.01 '*' 0.05 '.' 0.1 '' 1.

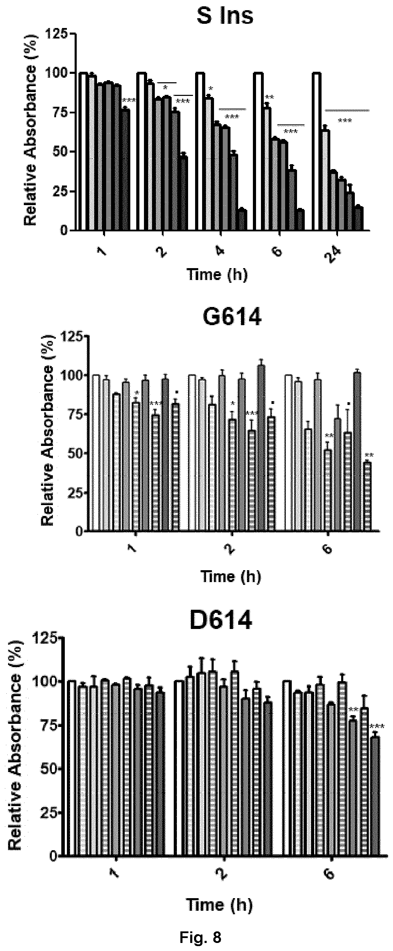

**Figure** 8. Gold Nanoparticles based sensors' functionality targeting the insertion region in S gene (S Ins) or the S mutation D614G. For the detection, RNA mimics of the different S regions were prepared and confronted to the complementary loops included in MB S Ins (grey), G614 (stripped greyscale) or D614 (grey) used for functionalize 23 nm AuNPs. Data are presented as mean ± SD (n=3) in terms of relative absorbance (%) vs negative control. The concentration of targets used (from left to right) was: 0, 10, 20, 30, 50 and 100 nM (for S Ins) or 0, 25, 50, 100 and 250 nM (for G614 and D614, both targets included in all experiments). Statistical analysis was performed using one-way ANOVA Tukey's test (pairwise comparison). Signif. codes: 0 '***' 0.001 '**' 0.01 '*' 0.05 '.' 0.1 '' 1.

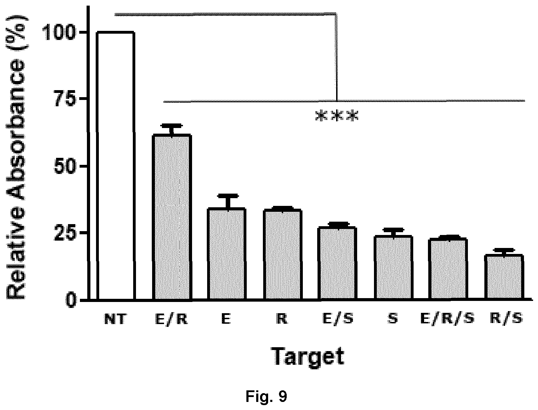

**Figure** 9. Gold Nanoparticles based sensors' in multiplexing experiments. For the detection, DNA mimics of the different genetic regions (S Ins, E or R) were incubated with 23 nm AuNPs with one, two or three MB at a time. Data corresponds to reactions using 50 nM DNA target concentration (final concentration, independently of the number of targets present in the mix) after 2h, and are presented as mean ± SD (n=3) in terms of relative absorbance (%) vs negative control. Statistical analysis was performed using one-way ANOVA with Tukey's test (pairwise comparison). Significance codes: 0 '***' 0.001 '**' 0.01 '*' 0.05 '.' 0.1 '' 1.

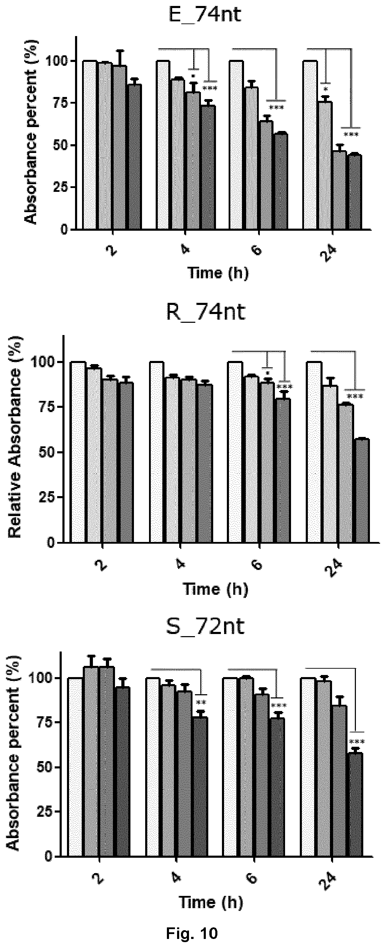

**Figure** 10. Gold Nanoparticles based sensors' functionality targeting the gene regions E, R or S mutation D614G. For the detection, ssDNA purified amplicons were prepared by RT-PCR followed by T7 exonuclease digestion and confronted to the complementary loops included in MB E, R or S G614 (loop 25nt) used functionalize 23 nm AuNPs. Data are presented as mean ± SD (n=3) in terms of relative absorbance (%) vs negative control (without target). The concentration of targets used (from left to right) were 0, 50 nM, 100 and 250 nM. Statistical analysis was performed using one-way ANOVA Tukey's test (pairwise comparison). Significance codes: 0 '***' 0.001 '**' 0.01 '*' 0.05 '.' 0.1 ' ' 1.

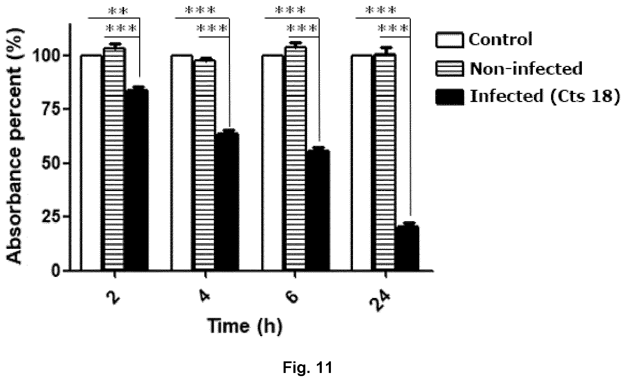

**Figure** 11. Gold Nanoparticles based sensors' functionality targeting the S gene mutation D614G region from COVID-19 patient's samples. For the detection, 250 nM ssDNA purified amplicon (infected patient) or and equivalent volume (non-infected patient) were prepared as described in the methods and used with the sensor S-G614. Data are presented as mean ± SD (n=3) in terms of relative absorbance (%) vs negative control (without target). Statistical analysis was performed using one-way ANOVA with Tukey's test (pairwise comparison). Significance codes: 0 '***' 0.001 '**' 0.01 '*' 0.05 '.' 0.1 '' 1.

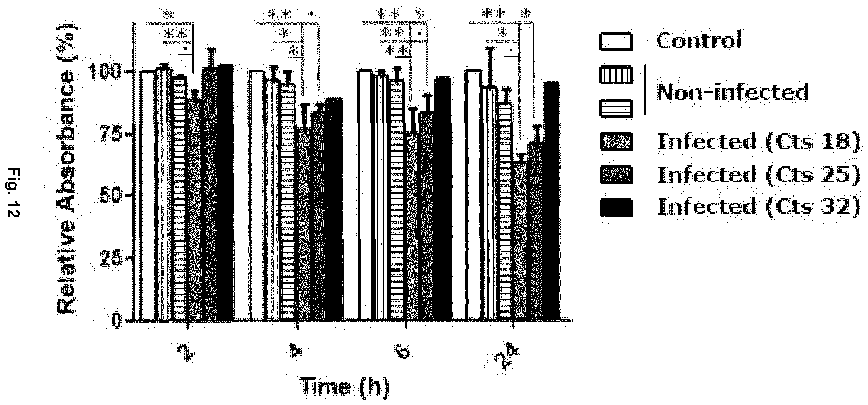

**Figure** 12. Gold Nanoparticles based sensors' functionality targeting the S gene mutation D614G region from non-infected and COVID-19 patient's samples with different viral loads. For the detection, crude reactions prepared by RT-PCR followed by T7 exonuclease digestion were confronted with the S G614 sensor. Data are presented as mean ± SD (n=3) in terms of relative absorbance (%) vs negative control (without target). Statistical analysis was performed using one-way ANOVA with Tukey's test (pairwise comparison). Significance codes: 0 '***' 0.001 '**' 0.01 '*' 0.05 '.' 0.1 '' 1.

## DETAILED DESCRIPTION OF THE INVENTION

The present description discloses a colorimetric test based on metal nanoparticles for the detection of SARS-CoV-2 nucleic acids which solves some of the disadvantages of the current gold standard detection methods, such as need for qualified personnel, sophisticated equipment, and expensive reagents. The methods herein presented are based on nanoparticles functionalized with oligonucleotides that are modified with a hydrophobic molecule, wherein said oligonucleotides target a region of SARS-CoV-2 genome.

Nanoparticle of the invention

In a first aspect, the invention relates to a metal nanoparticle bound to an oligonucleotide, from here onwards the nanoparticle of the invention, wherein said oligonucleotide comprises a first nucleotide region, a second nucleotide region and a third nucleotide region, in that order, wherein said first and third regions are at least partially complementary such that they can give rise to a hairpin-type structure by means of hybridization, wherein said second polynucleotide region is at least partially complementary to a region of the SARS-CoV-2 genome and wherein said oligonucleotide is modified with a hydrophobic molecule at the end opposite the end bound to the metal nanoparticle.

The term "nanoparticle" is used to designate colloidal systems of the spherical type, rod type, polyhedron type, etc., or similar shapes, having a size less than 1 micrometer ($\mu$m), which are individually found or are found forming organized structures (dimers, trimers, tetrahedrons, etc.), dispersed in a fluid (aqueous solution). In a particular embodiment, the nanoparticles suitable for putting the invention into practice have a size less than 1 $\mu$m, generally comprised between 1 and 999 nanometers (nm), typically between 5 and 500 nm, preferably between about 10 and 150 nm. In a particular embodiment, the nanoparticle of the invention typically have a mean particle diameter ranging from 2 to 50 nm, preferably from 5 to 45 nm, more preferably of about 26 nm. The mean particle diameter is the maximum mean particle dimension, with the understanding that the particles are not necessarily spherical. In another particular embodiment the nanoparticle of the invention have a diameter of about 10 to about 40 nm, preferably of about 15 to 38 nm, more preferably of about 20 to about 25 nm. The shape of said nanoparticles can widely vary; advantageously, said nanoparticles will adopt any optically efficient shape such as spheres, rods, stars, cubes, polyhedrons or any other variant as well as complex associations of several particles; in a particular embodiment, the shape of the nanoparticles for putting the invention into practice is spherical or substantially spherical. The shape can be suitably evaluated by conventional light or by means of electron microscopy techniques.

The term "metal nanoparticle" as used herein refers to a nanoparticle comprising a metal and showing the optical property known as the surface plasmon phenomenon, i.e., a plasmonic metal. This phenomenon consists of the collective vibration of the electrons of the metal surface, producing an absorption band located in the ultraviolet-visible spectrum (typical of the metal and of the size of the nanoparticles) at the wavelength where the resonance condition occurs in said electrons. Methods and techniques to determine the surface plasmon of a metal are described further below in the present description. As defined herein, a "plasmonic metal" is a metal characterized by showing the property of optics known as the surface plasmon phenomenon. The variation of the plasmonic response is particularly evident when several nanoparticles are located close to one another, given that this causes the coupling of their respective near fields, generating a new surface plasmon. In a particular embodiment of the nanoparticle of the invention, said metal is selected from the group consisting of gold, silver, copper, aluminum, platinum, iron, cobalt, palladium and combinations thereof. In an even more particular embodiment, the metal forming said nanoparticle is gold.

The nanoparticle according to the present invention is bound to an oligonucleotide. As it is used herein, the term "oligonucleotide" refers to a nucleic acid preferably of at least 10 nucleotides, preferably at least 16 nucleotides, preferably at least 20 nucleotides, preferably at least 25 nucleotides, and preferably not more than 100 nucleotides, including both polyribonucleotides and polydeoxyribonucleotides or combinations thereof. The term "oligonucleotide" also refers to molecules formed by conventional nucleotides bound by conventional phosphodiester bonds as well as variants thereof, including modifications in the purine or pyrimidine residues and modifications in the ribose or deoxyribose residues designed for increasing biological stability of the oligonucleotide or for stabilizing the physical stability of the hairpin-shaped structure. Examples of modified nucleotides that can be used in the present invention include, but are not limited to, nucleotides having at position 2' of the sugar a substituent selected from the fluoro, hydroxyl, amino, azido, alkyl, alkoxy, alkoxyalkyl, methyl, ethyl, propyl, butyl group or a functionalized alkyl group such as ethylamino, propylamino and butylamino. Alternatively, the alkoxy group is methoxy, ethoxy, propoxy or a functionalized alkoxy group according to the formula $O(CH_2)_q$-R, where q is 2 to 4 and R is an amino, methoxy or ethoxy group. Suitable alkoxyalkyl groups are methoxyethyl and ethoxyethyl. Oligonucleotides in which different nucleotides contain different modifications at position 2' are also part of the invention. Alternatively or additionally, the oligonucleotides of the invention can contain modified bonds such as phosphodiester-, phosphotriester-, phosphorothioate-, phosphorodithioate-, phosphoroselenoate-, phosphorodiselenoate-, phosphoroanilothioate-, phosphoramidate-, methylphosphonate-, boranephosphonate-type bonds as well as combinations thereof, or they are peptide nucleic acids (PNAs), in which the different nucleotides are bound by amide bonds. The chemical synthesis of nucleic acids is a well-known technique and it can be performed, for example, by means of using an automatic DNA synthesizer (such as commercially available Bioautomation synthesizers, for example). Said oligonucleotide can be synthesized by methods known in the art as the solid-phase phosphoramidite method, the triester method or the phosphorothioate method. For example, said oligonucleotide can be synthesized synthetized in a H8 DNA/RNA synthesizer (K&A Laborgeraete), as shown in Example section, Materials and Methods - Oligonucleotides design subsection of the present application. Other synthesis include solid supports for oligonucleotide synthesis that are well known by the person skilled in the art include, but are not limited to, inorganic substances such as silica, porous glass, aluminosilicates, borosilicates, metal oxides, etc. Alternatively, said solid support can be an organic substance, such as a cross-linked polymer, such as polyamide, polyether, polystyrene, etc. The preferred solid support for use in the synthesis of the oligonucleotide forming the nanoparticle according to the present invention is controlled porous glass (CPG).

The oligonucleotide comprises a first nucleotide region, a second nucleotide region and a third nucleotide region, in that order, wherein said first and third regions are at least partially complementary such that they can give rise to a hairpin-type structure by means of hybridization.

As it is used herein, the term "partially complementary" refers to said regions having a degree of complementarity that is sufficient for specific hybridization. Absolute complementarity between said first and third regions of the oligonucleotide is not required although it is preferred. In a particular embodiment of the nanoparticle of the invention, the degree of complementarity between the first and third regions of the oligonucleotide is at least 10%, at least 20%, at least 30%, at least 40%, at least 50%, at least 60%, at least 70%, at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, at least 99%, more preferably the degree of complementarity between the first and third regions is 100%. The degree of complementarity between both regions is determined by means of comparing the order of the nitrogen bases forming said first and third regions without taking into account structural differences between said first and third regions, provided that said structural differences do not prevent the formation of hydrogen bridges between the complementary bases. The degree of complementarity between said first and third regions can also be determined in terms of the number of unpaired bases present between said first and third regions. As the person skilled in the art will understand, the hybridization capacity between said first and third regions will depend on the degree of complementarity between both regions and on the length of the oligonucleotide.

In a particular embodiment of the nanoparticle of the invention, the first and third regions of said oligonucleotide forming the nanoparticle of the invention are of equal length, i.e., they comprise the same number of nucleotides. In another particular embodiment, said first and third regions are of different lengths, wherein the first region comprises more nucleotides than the third region, or wherein the third region comprises more nucleotides than the first region. In another particular embodiment of the nanoparticle of the invention the first and third regions comprise each 2 to 50 nucleotides, preferably 2 to 40 nucleotides, more preferably 2 to 30 nucleotides, more preferably 2 to 20 nucleotides, yet more preferably 2 to 10 nucleotides, even more preferably 4 to 8 nucleotides. In another embodiment of the nanoparticle of the invention the first and third region comprise each 2, 3, 4, 5, 6, 7, 8, 9, 10, 11, 12, 13, 14, 15, 20, 25, 30, 40 or 50 nucleotides, preferably 5 nucleotides.

In a particular embodiment of the nanoparticle of the invention, the first and third region comprise or consist of, respectively, the sequences CGGC and GCCG. In another embodiment, the first and third region comprise or consist of, respectively, the sequences CGGCC and GGCCG. In another embodiment, the first and third region comprise or consist of, respectively, the sequences GCGGCC and GGCCGC. In a particular embodiment, the first and third region comprise or consist of, respectively, the sequences CAGTC and GACTG.

Hybridization between the first and third regions of said oligonucleotide forming the nanoparticle of the invention gives rise to the formation of a hairpin-type structure. As it is used herein, a "hairpin-type structure" or "hairpin loop" refers to a secondary structure formed by an oligonucleotide comprising a stem region and a loop region, wherein said stem region is formed by a sequence of a double-stranded oligonucleotide formed by the hybridization between two or more nucleotide regions which are partially complementary to one another, and wherein said loop region is formed by a single-stranded sequence comprising unpaired bases. In the present invention, hybridization between the first and third regions of the oligonucleotide forming the nanoparticle of the invention gives rise to said stem region, and the second region of said oligonucleotide gives rise to the loop region of the hairpin-type structure. In a particular embodiment of the nanoparticle of the invention, the stem region comprises from 2 to 50 paired nucleotides, more preferably 3 paired nucleotides, 4 paired nucleotides, 5 paired nucleotides, 6 paired nucleotides, 7 paired nucleotides, 8 paired nucleotides, 9 paired nucleotides, 10 paired nucleotides, 15 paired nucleotides, 20 paired nucleotides, 30 paired nucleotides, 40 paired nucleotides or 50 paired nucleotides; and the loop region preferably comprises 3 nucleotides, 4 nucleotides, 5 nucleotides, 6 nucleotides, 7 nucleotides, 8 nucleotides, 9 nucleotides, 10 nucleotides, 15 nucleotides, 20 nucleotides, 30 nucleotides, 40 nucleotides, 50 nucleotides or more.

The oligonucleotide forming the nanoparticle of the invention comprises a second nucleotide region flanked by said first and third nucleotide regions wherein said second polynucleotide region is at least partially complementary to a region of the SARS-CoV-2 genome. The term "SARS-CoV-2", as used herein, refers to a virus characterized by the genome deposited on GenBank under accession code NC_045512 (version 2 of July 18th, 2020) or as shown in the GenBank database under accession number MN908947 (version 3 of March 18th, 2020) and derivatives thereof having at least 80%, preferably 85%, preferably 88%, preferably 90%, preferably 91%, preferably 92%, preferably 93%, preferably 94%, preferably 95%, preferably 96%, preferably 97%, preferably 98%, preferably 99%, preferably 99.5%, preferably 99.8%, preferably 99.9% or 99.99% sequence identity over the entire genome nucleotide sequence. In a particular embodiment of the nanoparticle of the invention the second region comprises 5, 6, 7, 8, 9, 10, 11, 12, 13, 14, 15, 20, 25, 30, 40, 50, 60, 70, 80, 90, 95 or 100 nucleotides. In a particular embodiment, said second region comprises a nucleotide sequence complementary to a target sequence.

It will be understood that the target sequence is defined as single-stranded RNA sequence or DNA sequence as the nanoparticles of the invention can be used for detecting the viral genome itself, in which the target sequence is an RNA sequence, as well as for detecting the products resulting from reverse transcription and optional amplification of the viral genome, in which case the target sequence will appear in the form of a DNA sequence.

Both GenBank database accession numbers NC_045512 (version 2 of July 18th, 2020) and MN908947 (version 3 of March 18th, 2020) represent the same genomic sequence of the SARS-CoV-2 genome. From here onwards only the accession number NC_045512 (version 2 of July 18th, 2020) will be used, even though the regions referenced are equally valid for the accession number MN908947 (version 3 of March 18th, 2020).

The SARS-CoV-2 genome is composed of a single-stranded positive-sense RNA that is around 30,000 nucleotides in length and encodes for 27 proteins, including the four structural proteins E (envelope, encoded by the E gene), N (nucleocapsid, encoded by the N gene), M (matrix, encoded by the M gene) and S (spike, encoded by the S gene), and the protein R (RNA-dependent RNA polymerase also known as RdRP, encoded by the ORF1ab gene). In a particular embodiment of the nanoparticle of the invention the second nucleotide region is at least partially complementary to a region of the SARS-CoV-2 genome selected from the group consisting of the E gene, the region of the ORF1ab gene which encodes for the R protein, the region of the S gene which encodes the region of the S protein containing the S1/S2 cleavage site and the region of the S gene which encodes the region of the S protein that contains the D residue at position 614.

The term "E gene" as used herein refers to the gene characterized by the nucleotide sequence between positions 26,245 and 26,472 of the SARS-CoV-2 genome as shown in the GenBank database under accession number NC_045512 (version 2 of July 18th, 2020) In a particular embodiment of the nanoparticle of the invention the second nucleotide region is at least partially complementary to a region of the E gene which corresponds to the region found between positions 26,245 and 26,472 of the SARS-CoV-2 genome as shown in the GenBank database under accession number NC_045512 (version 2 of July 18th, 2020). In another particular embodiment, the second nucleotide region is at least partially complementary to a region of the E gene which comprises the sequence SEQ ID NO: 1 or SEQ ID NO: 2.

SEQ ID NO: 1
ACACTAGCCATCCTTACTGCGCTTCG
SEQ ID NO: 2
ACACUAGCCAUCCUUACUGCGCUUCG

The expression "region of the ORF1ab gene which encodes for the R protein" as used herein refers to the gene region characterized by the nucleotide sequence between positions 13,442 and 16,236 of the SARS-CoV-2 genome as shown in the GenBank database under accession number NC_045512 (version 2 of July 18th, 2020). In a particular embodiment of the nanoparticle of the invention the second nucleotide region is at least partially complementary to a region of the ORF1ab gene which encodes for the R protein which corresponds to the region found between positions 13,442 and 16,236 of the SARS-CoV-2 genome as shown in the GenBank database under accession number NC_045512 (version 2 of July 18th, 2020). In another particular embodiment, the second nucleotide region is at least partially complementary to a region of the ORF1ab gene which encodes for the R protein consisting of sequence SEQ ID NO: 3 or SEQ ID NO: 4.

SEQ ID NO: 3
CAGGTGGAACCTCATCAGGAGATG
SEQ ID NO: 4
CAGGUGGAACCUCAUCAGGAGAUG

The term "S gene" as used herein refers to the gene characterized by the nucleotide sequence between positions 21,563 and 25,384 of the SARS-CoV-2 genome as shown in the GenBank database under accession number NC_045512 (version 2 of July 18th, 2020). In a particular embodiment of the nanoparticle of the invention the second nucleotide region is at least partially complementary to a region of the S gene which corresponds to the region found between positions 21,563 and 25,384 of the SARS-CoV-2 genome as shown in the GenBank database under accession number NC_045512 (version 2 of July 18th, 2020). The S gene of SARS-CoV-2 encodes for the spike protein characterized by the amino acid sequence as shown in the UniProt database under accession number PODTC2 of April 22, 2020. The spike protein is the responsible for allowing the virus to attach to and fuse with the membranes of a host cell. In order to fuse with the membrane the spike protein has to be cleaved at the S1/S2 and S2' cleavage sites. In particular embodiment of the nanoparticle of the invention the second nucleotide region is at least partially complementary to a region of the S gene which encodes the S1/S2 cleavage site in the spike protein which corresponds to the region found between positions 23,595 and 23,621 of the SARS-CoV-2 genome as shown in the GenBank database under accession number NC_045512 (version 2 of July 18th, 2020). In particular embodiment of the nanoparticle of the invention the second nucleotide region is at least partially complementary to a region of the S gene which encodes the S1/S2 cleavage site in the spike protein consisting of sequence of SEQ ID NO: 5 or SEQ ID NO: 6.

SEQ ID NO: 5
CTAATTCTCCTCGGCGGGCACGTAGTG

SEQ ID NO: 6
CUAAUUCUCCUCGGCGGGCACGUAGUG

Several variants of SARS-CoV-2 are characterized in part by single nucleotide polymorphisms in the S gene, which lead to modifications in the spike protein (Table 1). In a particular embodiment of the nanoparticle of the invention the second nucleotide region is at least partially complementary to a region of the S gene which encodes the region of the S protein which contains at least one of the mutations shown in Table 1 and is capable of hybridizing to the region of the S gene which encodes the region of the S protein which contains at least said mutation. In a particular embodiment of the nanoparticle of the invention the second nucleotide region is at least partially complementary to a region of the S gene which encodes the region of the S protein which contains the D residue at position 614 and is capable of specifically hybridizing to the region of the S gene which encodes the region of the S protein which contains a D614G mutation. In particular embodiment the second nucleotide region is at least partially complementary to a region of the S gene which encodes the region of the S protein which contains the D residue at position 614 which corresponds to the region found between positions 23,391 and 23,415 of the SARS-CoV-2 genome as shown in the GenBank database under accession number NC_045512 (version 2 of July 18th, 2020). In particular embodiment the second nucleotide region is at least partially complementary to a region of the S gene which encodes the region of the S protein which contains the D residue at position 614 which comprises the sequence of SEQ ID NO: 7, SEQ ID NO: 8, SEQ ID NO: 9, SEQ ID NO: 10, SEQ ID NO: 11 or SEQ ID NO: 12.

SEQ ID NO: 7
TATCAGGATGTTAAC

SEQ ID NO: 8
UAUCAGGAUGUUAAC

SEQ ID NO: 9
TATCAGGGTGTTAAC

SEQ ID NO: 10
UAUCAGGGUGUUAAC

SEQ ID NO: 11
TTCTTTATCAGGGTGTTAACTGCAC

SEQ ID NO: 12
UUCUUUAUCAGGGUGUUAACUGCAC

*Table 1: List of mutations in the spike protein that are present in SARS-CoV-2 variants*

| Residue | Position | New residues(s) | Mutations |
|---------|----------|-----------------|-----------|
| N | 501 | Y, T, S | N501Y, N501S |
| E | 484 | K | E484K |
| H | 69 | - | H69- |
| Q | 677 | H, P | Q677H, Q677P |
| Y | 453 | F | Y453F |
| K | 417 | N | K417N |
| H | 655 | Y | H655Y |

(continued)

| Residue | Position | New residues(s) | Mutations |
|---------|----------|-----------------|-----------|
| P | 681 | H | P681H |
| Y | 144 | - | Y144- |
| L | 18 | F | L18P, L18F |
| S | 477 | N | S477N |
| D | 614 | G | D614G |

In a particular embodiment of the nanoparticle of the invention the oligonucleotide comprises a sequence selected from the group consisting of SEQ ID NO: 13 to SEQ ID NO: 22.

*Table 2: Sequences of oligonucleotides of the nanoparticle of the invention*

| Oligonucleotide sequences | |
|---|---|
| SEQ ID NO: 13 | TTTTTCGGCCGAAGCGCAGTAAGGATGGCTAGTGTGCCG |
| SEQ ID NO: 14 | TTTTTCGGCCCGAAGCGCAGTAAGGATGGCTAGTGTGGCCG |
| SEQ ID NO: 15 | TTTTTGCGGCCCGAAGCGCAGTAAGGATGGCTAGTGTGGCCGC |
| SEQ ID NO: 16 | TTTTTCGGCGCATCTCCTGATGAGGTTCCACCTGGCCG |
| SEQ ID NO: 17 | TTTTTCGGCCGCATCTCCTGATGAGGTTCCACCTGGGCCG |
| SEQ ID NO: 18 | TTTTTGCGGCCGCATCTCCTGATGAGGTTCCACCTGGGCCGC |
| SEQ ID NO: 19 | TTTTTCGGCCC**ACTACGTGCCCGCCGAGGAGAATTAG**GGCCG |
| SEQ ID NO: 20 | TTTTTCAGTCGTTAACATCCTGATAGACTG |
| SEQ ID NO: 21 | TTTTTCAGTCGTTAACACCCTGATAGACTG |
| SEQ ID NO: 22 | TTTTTCAGTCGTGCAGTTAACACCCTGATAAAGAAGACTG |

The oligonucleotide forming part of the metal nanoparticle of the invention has modifications at the 3' and 5' ends. At one of its ends, the oligonucleotide is modified with a suitable group for the binding of the oligonucleotide to the metal nanoparticle. As it is used herein, the term "suitable group for the binding of the oligonucleotide to the metal nanoparticle" refers to a group capable of reacting with the metal atoms of the nanoparticle allowing the binding between the oligonucleotide and the nanoparticle. In a particular embodiment of the nanoparticle of the invention the oligonucleotide is bound to the nanoparticle by a group selected from a group containing a sulfur atom, a phosphorus atom or a nitrogen atom.

In a particular embodiment of the nanoparticle of the invention, said oligonucleotide is modified with a group containing a sulfur, a phosphorus or a nitrogen atom at one end and with a hydrophobic molecule at the opposite end.

In a particular embodiment of the nanoparticle of the invention the oligonucleotide is bound to the nanoparticle through a sulfur atom forming part of a group containing the sulfur atom. The term "group containing the sulfur atom" refers to a set of atoms containing sulfur which are bound to a carbon chain by means of a covalent bond. In a particular embodiment of the nanoparticle of the invention the oligonucleotide is bound to the nanoparticle through a phosphorus atom forming part of a group containing the phosphorus atom. The term "group containing the phosphorus atom" refers to a set of atoms containing phosphorus which are bound to a carbon chain by means of a covalent bond. In a particular embodiment of the nanoparticle of the invention the oligonucleotide is bound to the nanoparticle through a nitrogen atom forming part of a group containing the nitrogen atom. The term "group containing the nitrogen atom" refers to a set of atoms containing nitrogen which are bound to a carbon chain by means of a covalent bond.

The group containing sulfur, nitrogen or phosphorus is preferably associated with the oligonucleotide in the same way that the different nucleotides are associated with one another, i.e., through oxygen at position 3' or at position 5' of the sugar (ribose or deoxyribose) ring. In a particular embodiment, the group containing a sulfur atom, the phosphorus atom or the nitrogen atom is bound to the nucleotide located at the 3' end of the oligonucleotide. In that case, the group containing sulfur, phosphorus or nitrogen is bound to the oxygen at position 5' of the ribose or deoxyribose ring. In another particular embodiment, the group containing the sulfur atom, the phosphorus atom or the nitrogen atom is bound to the 5' end of the oligonucleotide. In that case, the group containing sulfur, phosphorus or nitrogen is bound to the oxygen at position 3' of the ribose or deoxyribose ring. Methods for obtaining oligonucleotides modified with groups containing sulfur, phosphorus or nitrogen are known in the state of the art and described in the next aspects of the invention.

In a particular embodiment of the nanoparticle of the invention, the binding between the oligonucleotide and the nanoparticle in the metal nanoparticle of the invention takes place through a sulfur-metal bond formed between the metal atoms located on the surface of the nanoparticle and the sulfur group which is bound to the oligonucleotide. In a particular embodiment of the nanoparticle of the invention, the sulfur-metal bond is formed by reaction between the metal atoms of the nanoparticle and a sulfur forming part of a functional group containing sulfur. In another particular embodiment, the sulfur-metal bond is a covalent bond. In a particular embodiment of the nanoparticle of the invention, the binding between the oligonucleotide and the nanoparticle in the metal nanoparticle of the invention takes place through a phosphorus-metal bond formed between the metal atoms located on the surface of the nanoparticle and the phosphorus group which is bound to the oligonucleotide. In a particular embodiment of the nanoparticle of the invention, the phosphorus-metal bond is formed by reaction between the metal atoms of the nanoparticle and a phosphorus forming part of a functional group containing phosphorus. In another particular embodiment, the phosphorus-metal bond is a covalent bond. In a particular embodiment of the nanoparticle of the invention, the binding between the oligonucleotide and the nanoparticle in the metal nanoparticle of the invention takes place through a nitrogen-metal bond formed between the metal atoms located on the surface of the nanoparticle and the nitrogen group which is bound to the oligonucleotide. In a particular embodiment of the nanoparticle of the invention, the nitrogen-metal bond is formed by reaction between the metal atoms of the nanoparticle and a nitrogen forming part of a functional group containing nitrogen. In another particular embodiment, the nitrogen-metal bond is a covalent bond. The terms "covalent", "covalent binding", "covalently bound" must be understood as the formation of a bond between two atoms or groups of atoms by sharing electron pairs.

In a particular embodiment of the nanoparticle of the invention, the sulfur-metal bond is formed by reaction between a sulfur atom of a functional group containing sulfur and a metal atom of the nanoparticle. The term "functional group containing sulfur" refers to a set of atoms containing sulfur which are bound to a carbon chain by means of a covalent bond which confer reactivity to the molecule containing them. In a particular embodiment, said functional group containing sulfur is selected from a thiol group, a dithiolane group and an isocyanide group.

As it is used herein, "thiol group" refers to a molecule formed by a sulfur atom and a hydrogen atom (SH). As it is used herein, an "isocyanide group" is an organic compound with the functional group R-N≡C. As it is used herein, "a dithiolane group" refers to a heterocyclic compound derived from cyclopentane containing sulfur formed by means of substituting two methyl groups ($CH_2$) with two thioether groups. The substituted methyl groups can be groups located at contiguous positions or at alternating positions in the heterocyclic ring. If two thioether groups substitute two methyl groups located at contiguous positions, then 1,2-dithiolane is obtained. If two thioether groups substitute two methyl groups occupying alternating positions in the heterocyclic molecule, then 1,3-dithiolane is obtained. In a preferred embodiment of the invention, said functional group containing sulfur is 1,2-dithiolane.

In a particular embodiment of the nanoparticle of the invention, the phosphorus-metal bond is formed by reaction between a phosphorus atom of a functional group containing phosphorus and a metal atom of the nanoparticle. The term "functional group containing phosphorus" refers to a set of atoms containing phosphorus which are bound to a carbon chain by means of a covalent bond which confer reactivity to the molecule containing them. In a particular embodiment, said functional group containing phosphorus is a phosphine group.

The term "phosphine group", as used herein refers to the class of organophosphorus compounds of substituted phosphanes-a class of phosphanes in which the hydrogen atoms have been replaced with organic derivative, having a general formula PR3.

In a particular embodiment of the nanoparticle of the invention, the nitrogen-metal bond is formed by reaction between a nitrogen atom of a functional group containing nitrogen and a metal atom of the nanoparticle. The term "functional group containing nitrogen" refers to a set of atoms containing nitrogen which are bound to a carbon chain by means of a covalent bond which confer reactivity to the molecule containing them. In a particular embodiment, said functional group containing nitrogen is selected from an amine group, an isocyanide group a pyridine group, a purine group and a pyrimidine group.

As it is used herein, an "amine group" refers to an organic chemical compound derived from ammonia in which one or several hydrogens of the ammonia molecule are substituted with other substituents or radicals.

In a more particular embodiment of the nanoparticle of the invention the oligonucleotide and the nanoparticle are connected by a spacer region. In another particular embodiment, the group containing the sulfur atom is bound to the oligonucleotide through a spacer sequence. In another particular embodiment, the group containing the phosphorus atom is bound to the oligonucleotide through a spacer sequence. In another particular embodiment, the group containing the nitrogen atom is bound to the oligonucleotide through a spacer sequence. The spacer sequence can be located at the 5' end of the oligonucleotide in the event that the group containing sulfur/phosphorus/nitrogen is located at the 5' end of the oligonucleotide, or it may be located at the 3' end of the oligonucleotide in the event that the group containing sulfur/phosphorus/nitrogen is located at the 3' end of the oligonucleotide. As it is used herein, "spacer sequence" refers to a nucleotide sequence to which binding with a molecule of interest is carried out, particularly with said functional group containing the sulfur/phosphorus/nitrogen atom. In a particular embodiment, the spacer sequence is formed by at least 2 nucleotides, by at least 3 nucleotides, by at least 4 nucleotides, by at least 5 nucleotides, by at least 6 nucleotides, by at least 7 nucleotides, by at least 8 nucleotides, by at least 9 nucleotides, by at least 10 nucleotides or more. In a particular embodiment of the nanoparticle of the invention, said spacer sequence is a polythymidine sequence. In a particular embodiment of the invention, the binding between said oligonucleotide and the group containing a sulfur atom, a nitrogen atom or a phosphorus atom is performed through a binding sequence formed by 5 thymidines. In another particular embodiment of the nanoparticle of the invention the spacer region further comprises an aliphatic carbon chain, wherein said aliphatic carbon chain has at least 2 carbon atoms (C2), at least 3 carbon atoms (C3), at least 4 carbon atoms (C4), at least 5 carbon atoms (C5), at least 6 carbon atoms (C6), at least 7 carbon atoms (C7), at least 8 carbon atoms (C8), at least 9 carbon atoms (C9), at least 10 carbon atoms (C10) or more. In a particular embodiment of the invention, the binding between said oligonucleotide and the group containing a sulfur, a phosphorus or a nitrogen atom is performed through a spacer region comprising an aliphatic carbon chain of 6 carbon atoms. In another particular embodiment of the nanoparticle of the invention the spacer region is formed by an aliphatic carbon chain bound to a nucleotide sequence, wherein the nucleotide sequence is bound to the oligonucleotide and the aliphatic carbon chain is bound to the group containing a sulfur, phosphorus or nitrogen atom bound to the nanoparticle, preferably wherein the aliphatic carbon chain has 6 carbon atoms and the nucleotide sequence is formed by 5 thymidines.

The term "aliphatic carbon chain" as used herein refers to hydrocarbons based on chains of carbon atoms. There are three types of aliphatic hydrocarbons. Alkanes are aliphatic hydrocarbons with only single covalent bonds. Alkenes are hydrocarbons that contain at least one C-C double bond, and alkynes are hydrocarbons that contain a C-C triple bond. In a preferred embodiment of the nanoparticle of the invention the spacer region comprises alkanes.

In a more particular embodiment of the nanoparticle of the invention, the hydrophobic group is bound to the oligonucleotide through a spacer sequence. The spacer sequence can be located at the 5' end of the oligonucleotide in the event that the hydrophobic group is located at the 5' end of the oligonucleotide, or it may be located at the 3' end of the oligonucleotide in the event that the hydrophobic group is located at the 3' end of the oligonucleotide.

In another particular embodiment of the nanoparticle of the invention, the oligonucleotide forming the metal nanoparticle of the invention is modified with a polyadenine sequence at the end opposite the end where it is modified with said hydrophobic molecule. Polyadenine sequences are capable of self-assembling by means of adsorption on the surface of metal particles. In a particular embodiment, said polyadenine sequence is formed by at least 5 adenines; in another preferred embodiment, said polyadenine sequence is formed by at least 10 adenines, at least 15 adenines, at least 20 adenines, at least 25 adenines, at least 30 adenines, at least 35 adenines, at least 40 adenines, at least 45 adenines, at least 50 adenines or more. The polyadenine sequence is preferably associated with the oligonucleotide in the same way that the different nucleotides are associated with one another, i.e., through the oxygen at position 3' or at position 5' of the sugar (ribose or deoxyribose) ring. In a particular embodiment of the nanoparticle of the invention, the polyadenine sequence is bound to the nucleotide located at the 3' end of the oligonucleotide. In another particular embodiment, the polyadenine sequence is bound to the 5' end of the oligonucleotide. The binding between said polyadenine sequence and the oligonucleotide forming the nanoparticle of the invention takes place through a phosphodiester bond. Methods for obtaining oligonucleotides modified with a polyadenine sequence are known in the state of the art and described further on in this description.

In a particular embodiment of the nanoparticle of the invention, the binding between the polyadenine sequence and the nanoparticle is produced by adsorption of said polyadenine sequence on the metal atoms of the nanoparticle. As it is used herein, the term "adsorption" refers to the process whereby atoms, ions or molecules are trapped or retained on the surface of a material.

The oligonucleotide forming the nanoparticle of the invention is modified with a hydrophobic molecule at the end opposite the end where it is modified with a group containing a sulfur atom, a phosphorus atom, a nitrogen atom or with a polyadenine sequence. The term "oligonucleotide modified with a hydrophobic molecule" refers to an oligonucleotide comprising, in addition to the nucleotides forming it, at least one hydrophobic molecule bound to the said oligonucleotide, preferably through one of the hydroxyl groups of the sugar molecules forming the nucleotides. As it is used herein, the term "hydrophobic molecule" refers to a molecule that is more soluble in organic solvents (for example, long-chain alcohols, esters, ethers, ketones and hydrocarbons) than in aqueous solutions. Basically, hydrophobicity occurs when the molecule referred to as hydrophobic is not capable of interacting with water molecules through ion-dipole interactions or by means of hydrogen bridges. Hydrophobicity of the "hydrophobic molecule" can be defined by the concentration ratio (partition coefficient) of the molecule distributed between an aqueous phase and an organic phase. Specifically, hydrophobicity can be defined by the partition coefficient log value (partition coefficient P (logP)) between the aqueous and organic phases (for definitions of the terms partition coefficient and partition ratio, refer to "Handbook of Chemistry, 5th ed., Basic II" [The Chemical Society of Japan ed., MARUZEN Co., Ltd., p. 168-177]). In the present invention, "logP" refers to a theoretical distribution coefficient calculated using a logP calculation program (for example, ACD/LogP, (version 9.0)), available from Advanced Chemistry Development, Inc., and an algorithm described in the ACD/LogP manual (2005) included therein. In the present invention, a "hydrophobic molecule" is defined as an organic molecule having a logP of 2 or more, preferably a logP of 7 or more, at pH 7. Specifically, the compound having a logP of 2 or more (i.e., the concentration ratio of the compound between the aqueous and organic phases is 1:100 or more) can be interpreted as having the property of forming aggregates through hydrophobic interactions with other molecules when they are distributed in a neutral aqueous solution.

The person skilled in the art will understand that the nanoparticles of the present invention are not limited in terms of the number of hydrophobic molecules modifying the oligonucleotide. Thus, it is possible to introduce more than one hydrophobic group in the oligonucleotide forming part of the nanoparticle. Thus, in particular embodiments, the oligonucleotide is modified with two, three, four, five, six, seven, eight, nine, ten or more hydrophobic groups.

In a particular embodiment of the nanoparticle of the invention, two or more nucleotides that are located at the 5' end of said oligonucleotide are modified with a hydrophobic molecule. In a preferred embodiment, three, four, five, six, seven, eight, nine, ten, fifteen or more nucleotides that are located at positions prior to the 5' end of the oligonucleotide are modified with a hydrophobic group.

In another particular embodiment of the nanoparticle of the invention, the oligonucleotide of the 5' end is modified with two, three, four, five, six, seven, eight, nine, ten or more hydrophobic groups. In another particular embodiment, three, four, five, six, seven, eight, nine, ten, fifteen or more nucleotides that are located at positions prior to the 5' end of the oligonucleotide are modified with two, two, three, four, five, six, seven, eight, nine, ten or more hydrophobic groups.

In a particular embodiment of the nanoparticle of the invention, said hydrophobic molecule is a lipid. As it is used herein, "lipid" refers to organic molecules that have a long-chain fatty acid or a hydrocarbon chain and are slightly soluble in water and readily soluble in organic solvents. In a more particular embodiment, said lipid is a steroid lipid. As it is used herein, "steroid lipid" refers to lipids derived from the nucleus of the hydrocarbon sterane (or cyclopentaneperhydrophenanthrene), being made up of four rings fused with functional hydroxyl and carbonyl groups. In a more particular embodiment, said steroid lipid is cholesterol or a cholesterol derivative. As it is used herein, "cholesterol" refers to a sterane molecule having two methyl radicals at positions $C_{10}$ and $C_{13}$, a branched aliphatic chain containing eight carbons at position $C_{17}$, a hydroxyl group at position $C_3$ and an unsaturation between the $C_5$ and $C_6$ carbons; where C refers to a carbon atom, and wherein the number represented next to said C refers to the specific position said carbon atom occupies in the molecule. The term "cholesterol derivatives" as used herein refers to compounds derived from the cholesterol and therefore, sharing a similar backbone structure. Examples of such compounds are bile salts, vitamin D, steroid hormones such as progesterones, glucocorticoids, mineralocosticoids, androgens, estrogens, estrogen, and aldosterone.

In another particular embodiment said lipid is a fatty acid. The "term" fatty acid as used herein refers to a carboxylic acid with a long aliphatic chain, which is either saturated or unsaturated. The terms "saturated" and "unsaturated" refer to the property of a chemical compound of resisting addition reactions, such as hydrogenation, oxidative addition and binding of a Lewis base. Said reactions are not possible with saturated compounds due to the fact that saturated compounds only have single bonds between carbon atoms. In a particular embodiment the fatty acid is a saturated fatty acid. In another particular embodiment the saturated fatty acid is selected from a group consisting of caprylic acid, capric acid, lauric acid, myristic acid, palmitic acid, stearic acid, arachidic acid, behenic acid, lignoceric acid and cerotic acid, preferably a palmitic acid. As used herein, "palmitic acid" refers to the hexadecanoic acid, with a chemical formula of $CH_3(CH_2)_{14}COOH$ (CAS number: 57-10-3). Palmitic acid is an amphipathic molecule, comprising a carboxylic acid portion (COOH) which is hydrophilic and a long hydrocarbon chain which is hydrophobic. In a preferred embodiment the oligonucleotide of the nanoparticle of the invention is modified with a palmitic acid wherein the carboxylic acid portion of the palmitic acid is bound to the oligonucleotide. Methods of modifying oligonucleotides with fatty acids are known in the field and known to the expert. An example of a method to modified a nucleotide with palmitic acid, without limitation, is the reaction of a single stranded nucleotide (4 nmol in 20 $\mu$L of water) with 40 nmol of palmitic acid *N*-hydroxysuccinimide ester dissolved in 10 $\mu$L *N,N*-dimethylformamide containing 0.7 $\mu$L of *N,N*-diisopropylethylamine in 100 $\mu$L of isopropanol/water mixture solutions for 12h at room temperature.

In a particular embodiment of the nanoparticle of the invention, said hydrophobic molecule is a polycyclic aromatic hydrocarbon, preferably a pyrene. The term "polycyclic aromatic hydrocarbon" or "PAHs" as used herein refers to a chemical compound containing only carbon and hydrogen that is composed of multiple aromatic rings. PAHs are uncharged, nonpolar molecules with distinctive properties due to the delocalized electrons in their aromatic rings. In a particular embodiment the polycyclic aromatic hydrocarbon is pyrene. As used herein the term pyrene is a chemical compound formed by four fused benzene rings, resulting in a flat aromatic system of chemical formula $C_{16}H_{10}$. Pyrenes can be linked to nucleotides via a short tether into the 2'-O-position of the oligonucleotide (Yamana et al., 1999, Acids Research, 27, 11, 1, 2387-2392).

In a particular embodiment of the nanoparticle of the invention, the group containing sulfur is bound to the 3' end of the oligonucleotide, in which case the hydrophobic molecule is bound to the 5' end of the oligonucleotide. In another particular embodiment, the group containing sulfur is bound to the 5' end of the oligonucleotide, in which case the hydrophobic molecule is bound to the 3' end of the oligonucleotide. In a particular embodiment of the nanoparticle of the invention, the group containing phosphorus is bound to the 3' end of the oligonucleotide, in which case the

EP 4 190 913 A1

hydrophobic molecule is bound to the 5' end of the oligonucleotide. In another particular embodiment, the group containing phosphorus is bound to the 5' end of the oligonucleotide, in which case the hydrophobic molecule is bound to the 3' end of the oligonucleotide. In a particular embodiment of the nanoparticle of the invention, the group containing nitrogen is bound to the 3' end of the oligonucleotide, in which case the hydrophobic molecule is bound to the 5' end of the oligonucleotide. In another particular embodiment, the group containing nitrogen is bound to the 5' end of the oligonucleotide, in which case the hydrophobic molecule is bound to the 3' end of the oligonucleotide. In another particular embodiment, the polyadenine sequence is bound to the 3' end of the oligonucleotide, in which case the hydrophobic molecule is bound to the 5' end of the oligonucleotide. In another particular embodiment, the polyadenine sequence is bound to the 5' end, in which case the hydrophobic molecule is bound to the 3' end of the oligonucleotide.

Methods for binding a hydrophobic molecule to an end of an oligonucleotide are well known in the state of the art and described in the context of the second aspect of the invention. The incorporation of the hydrophobic group into the oligonucleotide is preferably carried out by means of using precursors of said groups activated with a phosphoramidite group, which allows the incorporation of the hydrophobic group by reacting the phosphoramidite group with a hydroxyl group in the sugar molecule of the oligonucleotide. In a preferred embodiment, the incorporation of the hydrophobic group is carried out by means of using the precursor cholesteryl-3-carboxamidohexyl-[(2-cyanoethyl)-(N,N-diisopropyl)]-phosphoramidite.

The nanoparticle of the invention has amphipathic characteristics, i.e., it has a watersoluble, hydrophilic region in its structure formed by the oligonucleotide forming part of said nanoparticle, and a hydrophobic region formed by the hydrophobic molecule bound to an end of the oligonucleotide. The amphipathicity of the molecule is variable depending on the folding state of the hairpin-shaped region. Thus, if the region with the hairpin structure is folded, the nanoparticle is more hydrophilic given that the loop of said region, which is hydrophilic, is exposed to the exterior. If the region with the hairpin structure is unfolded, the nanoparticle is more hydrophobic given that the hydrophobic group is exposed to the exterior of the nanoparticle. In a particular embodiment of the nanoparticle of the invention, the nanoparticles are stabilized so that they do not substantially aggregate when the oligonucleotide is forming a hairpin structure by the annealing of the first and third regions. The expression "nanoparticles are stabilized" as used herein refers to the process of avoiding nanoparticle aggregation by changing the net charge of the nanoparticle by steric and/or electrostatic effects. "Steric effects" or "steric stabilization" as used herein refer to the use of capping agents to act as a barrier to avoid the attractive forces that occur with nanoparticles when in close proximity and therefore avoiding aggregation of nanoparticles. Examples of such capping agents are material surfactants, synthetic polymers, biopolymers, amino acids, proteins, cyclodextrins and citrate. In a particular embodiment the nanoparticles are stabilized by citrate. The term "electrostatic effects" as used herein refers to the introduction of ions (electrolytes) in the solution as to contract the forces present in the NPs surface and attain an equilibrium whereby the nanoparticles do not aggregate. Methods and techniques for stabilization of nanoparticles are widely known in the field and available in the literature (Zhao et al., 2013, Coordination Chemistry Reviews, 257, 638-665). The expression "the oligonucleotide is forming a hairpin structure by the annealing of the first and third regions" refers to the secondary structure formed by the oligonucleotide of the nanoparticle of the invention, wherein the first and third regions come together and due to their base complementarity, i.e., they base-pair leading the second region to form a loop of non-annealed nucleotide sequence in between the first and third regions. This structure is also called a stem loop.

Process for obtaining the nanoparticle of the invention

In a second aspect, the invention relates to a process for obtaining the nanoparticle of the invention, from here onwards the process of the invention, which comprises:

(i) contacting metal nanoparticles with an oligonucleotide, wherein the oligonucleotide comprises a first, second and third region according to the invention and wherein the oligonucleotide is modified with a group which is capable of reacting with the surface of the nanoparticle and; and
(ii) maintaining the nanoparticles under suitable conditions for the formation of a bond between said oligonucleotide and the metal nanoparticle.

All terms and expressions previously defined in this description in relation to previous aspects have the same meaning, except where differently stated, for the current aspect and its embodiments, and the previous definitions are equally valid.

The first step of the process for obtaining the metal nanoparticles of the invention comprises contacting metal nanoparticles with an oligonucleotide, wherein said oligonucleotide comprises a first, a second and a third nucleotide region according to the invention.

The metal nanoparticles used in the first step of said method can be obtained using any conventional methodology. The general method for obtaining metal nanoparticles is based on the preparation of an aqueous solution comprising one or more metal salts to which there is added a reducing agent capable of reducing the metal salt cation to the metal state. In a particular embodiment of the process of the invention, the method for obtaining the nanoparticles relates to obtaining metal nanoparticles wherein the metal is selected from the group consisting of gold, silver, copper, aluminum, platinum, iron, cobalt, palladium and combinations thereof. In another particular embodiment of the process of the invention, the method for obtaining the nanoparticles of the invention relates to obtaining gold nanoparticles. In a particular embodiment of process of the invention, the metal salts used for obtaining said nanoparticles comprise gold salts, more preferably HAuCl4. The reducing agents capable of reducing the metal salt cation to the metal state are known by the person skilled in the art (Bradley, J. S., Schmid, G., Talapin, D. V., Shevchenko, E. V. and Weller, H. (2005) Syntheses and Characterizations: 3.2 Synthesis of Metal Nanoparticles, in Nanoparticles: From Theory to Application (ed. G. Schmid), Wiley-VCH Verlag GmbH & Co. KGaA, Weinheim, FRG. doi: 10.1002/3527602399.ch3b. ISBN: 9783527305070). Hydrides, citrates, alcohols, reducing polymers, hydrazine, hydroxylamine or their derivatives and mixtures thereof can be used as reducing agents. In a particular embodiment, the reducing agent used is trisodium citrate.

Once the metal nanoparticles in solution are obtained, they are collected, preferably by means of filtration or centrifugation, and optionally purified. The purification of the nanoparticles can be carried out by means of processes of washing/centrifuging in a suitable solvent, preferably water, or by means of dialysis. The nanoparticles thus obtained can be detected by UV-Vis spectroscopy, transmission electron microscopy (TEM) or Raman spectroscopy. Likewise the, same techniques can be used to determine the diameter of the nanoparticles. In a particular embodiment the nanoparticle of the invention have a diameter of about 10 to about 40 nm, preferably of about 15 to 38 nm, more preferably of about 20 to about 25 nm. In a particular embodiment, the nanoparticles of the invention are detected by means of TEM and/or UV-Vis spectroscopy at 520 nm. The concentration of said nanoparticles can be estimated by means of any technique suitable for that purpose. By way of illustration, an estimation of the concentration of the obtained nanoparticles can be determined by means of the Lambert-Beer law or by means of determining the molar extinction coefficient, which is a parameter that defines the radiation absorbed by a substance at a certain wavelength per molar concentration.

If desired, the method for obtaining the nanoparticles of the invention can be carried out by means of the method of reduction with citrate illustrated in the Examples section, subsection Materials and Methods - Synthesis of gold nanoparticles.

The first step of the method for obtaining the nanoparticles of the invention comprises contacting metal nanoparticles obtained by means of any suitable method with an oligonucleotide. Said oligonucleotide has already been described in relation to the nanoparticles of the invention and all the embodiments relating to the nanoparticles of the invention aspect are equally valid to the method for obtaining nanoparticles of the invention.

In a particular embodiment of the process of the invention, the oligonucleotide being contacted with the nanoparticle is modified at one of its ends with a functional group containing a sulfur atom selected from a thiol group, a dithiolane group, and an isocyanide group. In an even more particular embodiment of the invention, said functional group containing a sulfur atom is a dithiolane group. In a particular embodiment of the invention, said functional group containing a sulfur atom is 1,2-dithiolane. In a particular embodiment of the process of the invention, the oligonucleotide being contacted with the nanoparticle is modified at one of its ends with a functional group containing a phosphorus atom from a phosphine group. In a particular embodiment of the process of the invention, the oligonucleotide being contacted with the nanoparticle is modified at one of its ends with a functional group containing a nitrogen atom selected from an amine group, an isocyanide group, a pyridine group, a purine group and a pyrimidine group.

In a particular embodiment of the process of the invention, the oligonucleotide forming the nanoparticle is modified at its 3' end with said functional group containing a sulfur atom, a phosphorus atom or a nitrogen atom. In another particular embodiment, the oligonucleotide forming part of the nanoparticle is modified at its 5' end with said functional group containing a sulfur atom, a phosphorus atom or a nitrogen atom.

The synthesis of an oligonucleotide modified at its 5' end or at its 3' end with a functional group containing a sulfur/phosphorus/nitrogen atom, for example with a dithiolane group, can be carried out by means of any suitable method known in the state of the art. Alternatively, the oligonucleotide can be acquired from a company (for example Sigma-Genosys), in which case it is important that the product is purified of other thiols, such as dithiothreitol (DTT) or dithioerythritol (DTE), which can be added as stabilizing agents. In such case, it is advisable to purify the oligonucleotide by means of any technique suitable for that purpose, such as for example, size exclusion chromatography, HPLC or electrophoresis in preparative gels.

If said oligonucleotide is synthesized using a DNA synthesizer, it can be carried out using the phosphoramidite method. Briefly, the synthesis method based on phosphoramidite chemistry is based on solid base synthesis of an oligonucleotide by means of the sequential addition of activated monomers. The activated monomers are protonated deoxyribonucleoside 3'-phosphoramidites. In a first step, the phosphorus atom at position 3' of the incoming element is bound to the oxygen atom at position 5' of the growing chain to form a phosphite triester. The hydroxyl group (OH) at position 5' of the activated monomer does not react because it is blocked by a dimethoxynitrile (DMT) protecting group, and the phosphoryl group at position 3' does not react because the beta-cyanoethyl group is bound thereto. Similarly, the amino groups of the purine and pyrimidine bases are blocked. Coupling is carried out under anhydrous conditions because the water reacts with phosphoramidites. In a second step, phosphite triester oxidizes to form a phosphate triester. In a third step, the DMT protecting group is detached from the OH at position 5' of the growing chain by means of adding a strong acid (such as trichloroacetic acid), leaving the protecting groups intact. At this point, the oligonucleotide has been elongated by one unit. To obtain an oligonucleotide phosphorylated at the 5', during the coupling cycle a phosphate containing group can be added, such as a derivative of sulfonyldethanol, which will react with the 5' OH group. Once bound, the phosphate group can be removed by reacting with aqueous ammonia which will lead to an acidic proton on the carbon atom adjacent to the sulfonyl group to be lost generating the 5'-phosphate oligonucleotide. The incorporation of the functional group containing sulfur or nitrogen can be carried out by means of adding to the synthesis reaction at the right time a monomer comprising a functional group with sulfur or with nitrogen activated with a phosphoramidite group. The monomer with the functional sulfur group or with the functional amino group will thus be incorporated in the molecule.

In a particular embodiment, the oligonucleotide is synthesized with the functional group containing sulfur, phosphorus or nitrogen bound to its 3' end. Said oligonucleotide can be generated by starting the synthesis on a solid support (for example CPG) modified with said group containing sulfur, such as a dithiolane group, or containing phosphorus, such as a sulfonyldethanol group, or containing nitrogen, such as aminoalkyl, for example, or alternatively by starting the synthesis on a conventional solid support and adding phosphoramidites modified with said dithiolane or sulfonyldethanol group, or adding 5'aminated oligomers as the first monomer in the sequence. Alternatively, the oligonucleotide can be synthesized with the dithiolane group bound to its 5' end. The synthesis of said oligonucleotide can be carried out by means of any technique suitable for that purpose, such as the phosphoramidite method, as discussed above. In any case, as discussed above, it is advisable to perform a final treatment step under acidic conditions to release the hydroxyl group from the DTT group.

Oligonucleotides modified at 3' or 5' with a functional group containing sulfur, such as a thiol group or a dithiolane group, can be obtained using other conventional techniques based on the phosphodiester or phosphotriester methods (Narang et al., 1979, Meth. Enzymol. 68: 90; Brown et al., Meth. Enzymol. 1979, 68, 109).

The oligonucleotide used in the first step of the process of the invention can be modified at both ends, 5' and 3', with various functional groups and hydrophobic moieties as well as have different first, second and third nucleotide regions which are composed of different sequences and/or target different regions of the SARS-CoV-2 genetic material. Said modifications and nucleotide regions have been previously defined in relation to the nanoparticle of the invention aspect and said embodiments are equally valid to the present inventive aspect concerning the process of the invention.

Once said oligonucleotide is obtained by means of any suitable method, the second step of the process of the invention comprises maintaining the nanoparticles under suitable conditions for the binding between the oligonucleotide and the metal of the nanoparticle. As the person skilled in the art will understand, in said step the oligonucleotide may be added to the suspension containing the metal nanoparticles, or alternatively, a suitable solution could be prepared from a suspension containing metal nanoparticles and a suitable solvent. The oligonucleotide that is bound to the nanoparticle of the invention will then be added to said solution.

In a particular embodiment, said conditions will be suitable conditions for forming a bond between the sulfur atom of the functional group containing sulfur bound to an end of the oligonucleotide and the metal of the nanoparticle. In a particular embodiment, said conditions will be suitable conditions for forming a bond between the phosphorus atom of the functional group containing phosphorus bound to an end of the oligonucleotide and the metal of the nanoparticle. In a particular embodiment, said conditions will be suitable conditions for forming a bond between the nitrogen atom of the functional group containing nitrogen bound to an end of the oligonucleotide and the metal of the nanoparticle.

In a particular embodiment, the sulfur-metal bond is formed by reaction between a sulfur atom of the functional group containing sulfur of the oligonucleotide and a metal atom of the nanoparticle. In a particular embodiment of the invention, said bond is formed between a sulfur atom of a dithiolane group bound to an end of the oligonucleotide and a gold atom of the nanoparticle. In a more preferred embodiment of the invention, said bond is formed between a sulfur atom of the dithiolane group bound to the 3' end of the oligonucleotide modified at the 5' end with a hydrophobic molecule, wherein said hydrophobic molecule is preferably a lipid, and more preferably cholesterol. In a particular embodiment, the phosphorus-metal bond is formed by reaction between a phosphorus atom of the functional group containing phosphorus of the oligonucleotide and a metal atom of the nanoparticle. In another particular embodiment of the invention, said bond is formed between a phosphorus atom of a phosphine group bound to an end of the oligonucleotide and a gold atom of the nanoparticle. In a more particular embodiment of the invention, said bond is formed between a phosphorus atom of the phosphine group bound to the 3' end of the oligonucleotide modified at the 5' end with a hydrophobic molecule, wherein said hydrophobic molecule is preferably a lipid, and more preferably cholesterol. In a particular embodiment, the nitrogen-metal bond is formed by reaction between a nitrogen atom of the functional group containing amino of the oligonucleotide and a metal atom of the nanoparticle. In another particular embodiment of the invention, said bond is formed between a nitrogen atom of an amine group bound to an end of the oligonucleotide and a gold atom of the nanoparticle. In a more preferred embodiment of the invention, said bond is formed between a nitrogen atom of the amine group bound to the 3' end of the oligonucleotide modified at the 5' end with a hydrophobic molecule, wherein said hydrophobic molecule is preferably a lipid, and more preferably cholesterol.

The binding between the oligonucleotide modified with a functional group of the ones described above and the metal atoms takes place spontaneously. Example subsection Materials and Methods - AuNP-based sensor preparation of the application shows suitable conditions for carrying out said conjugation. By way of illustration, said reaction can be carried out by incubating in a 0.3 M sodium chloride solution at a concentration of about $2.7 \times 10^8$ M-1 cm-1 gold nanoparticles, obtained by any of the aforementioned methods, for example obtained by means of the citrate sodium method, with the modified oligonucleotide at a suitable concentration, preferably in excess, of about 10 $\mu$M for a suitable time interval so that the nanoparticles are coated by said oligonucleotide, for example for 16 hours. Said reaction is carried out under mild temperature and pH conditions, such as room temperature and physiological pH. After the reaction has occurred, the obtained nanoparticles are purified by means of three centrifugation cycles.

In another particular embodiment, the second step of the process of the invention relates to suitable conditions so that the metal of the nanoparticle adsorbs the polyadenine sequence. The conjugation between the oligonucleotide modified with a polyadenine sequence and the metal nanoparticle can be carried out using conditions similar to those described for conjugating the metal nanoparticles with an oligonucleotide modified with a functional group as previously described.

Method for detecting the presence of SARS-CoV-2 genetic material in a sample

The inventors have shown that the nanoparticles of the invention can be used to detect the genetic material of SARS-CoV-2 in a sample which contains complementarity with the loop region of the oligonucleotides forming part of the nanoparticles of the invention.

Therefore, another aspect of the present invention relates to a method for detecting the presence of a SARS-CoV-2 genetic material in a test sample, from here onwards the method of the invention, which comprises:

(i) contacting a nanoparticle according to the nanoparticle of the invention with said sample wherein said contacting is carried out under conditions that allow hybridizing said region of the oligonucleotide of the nanoparticle with the nucleic acid of said sample; and
(ii) detecting aggregation of the nanoparticles, wherein an increase in aggregation with respect to a control sample indicates that the test sample contains SARS-CoV-2 genetic material.

All terms and expressions previously defined in this description in relation to previous aspects, have the same meaning, except where otherwise stated, for the current aspect and its embodiments, and the previous definitions are equally valid. Likewise, all the embodiments relating to the nanoparticles of the invention aspect are equally valid to the method of the invention.

In the first step, the method of the invention comprises contacting the nanoparticle of the invention with the test sample of interest. As used herein the term "test sample" refers to a portion, a small quantity or part of something which is representative of the whole. In a particular embodiment of the method of the invention the test sample is a biological sample. As used herein the term "biological sample" refers to material obtained from a biological subject or source. The biological sample may contain any type of material which is suitable to perform the first method of the invention. In a particular embodiment the biological sample may be pretreated before performing the first method of the invention. Examples of said pretreatments are washing, centrifuging, concentrating, lysing, separation of types of tissue and/or cells, etc. These methods and their application are well known to the skilled person in the field. In another particular embodiment of the method of the invention the biological sample is selected from a group formed by a biological fluid, a cell lysate or a tissue lysate. The term "biological fluid" as used herein refers to any liquid which can be isolated from a subject. In a particular embodiment the biological fluid is selected from a group consisting of sputum, oropharyngeal washings, nasal aspirates, bronchoalveolar washings, blood, pleural fluid, amniotic fluid and nasopharyngeal swabs. In another particular embodiment the biological sample is a nasopharyngeal swab. The term "cell lysate" and "tissue lysate" refers to samples which were pretreated to disrupt and rupture the cellular membranes of the cells and tissues in order to obtain access to or release to the external environment the content of their cytosol. Said lysis can be achieved using several methods and techniques well known in the art such as, and without limitation, chemical lysis (e.g., detergents such as odium dodecyl sulphate (SDS), Triton X-100), acoustic lysis through ultrasonic waves and mechanical lysis (e.g., shearing through capillary tips).

Also in the first step, the oligonucleotide forming part of the nanoparticle is capable of hybridizing with the genetic material of said sample. The term "genetic material" as used herein is a synonymous of the term "nucleic acids" and both are used interchangeably, such as, without limitation, DNA, RNA, mRNA, complementary DNA (cDNA), etc.

The use of RNA in the method of the invention may imply the extraction of said RNA from cell and/or tissues. RNA extraction can be performed by any of the methods known by the person skilled in the art, including, without being limited to, Trizol®, guanidinium salts, phenol, chloroform, etc. Said methods can be found, for example, in Sambrook et al., 2001. "Molecular cloning: a Laboratory Manual", 3rd ed., Cold Spring Harbor Laboratory Press, N.Y., Vol. 1-3. There are also commercial kits that allow extracting RNA from a sample, such as the Qiagen® RNA extraction kit, for example. As the person skilled in the art knows, maximum precautions must be taken when working with RNA to avoid contaminations with RNases and RNA degradation. From the RNA extracted, cDNA may be obtained by reverse transcription, explained in detail further along in this description.

The nanoparticle of the invention and processes suitable for obtaining it are defined in the context of the previous aspects of the present invention. Said first step must take place under conditions that allow hybridizing said oligonucleotide with the target nucleic acid of the sample. As it is used herein, the term "hybridization" refers to the formation of a duplex-type structure by two nucleic acids due to complementary base pairing. Hybridization can occur between completely complementary nucleic acid chains or between "substantially complementary" nucleic acid chains containing small unpaired regions. In the context of the present invention, "specific hybridization" is understood as the binding, hybridizing or duplex-forming capacity of the second region of the oligonucleotide forming part of the nanoparticle of the invention with the target sequence to be detected such that said target nucleic acid to be detected can be identified or be distinguished from other components of a mixture (for example, cell extracts, genomic DNA, etc.). The oligonucleotide of the nanoparticle of the method of the invention is defined in the first aspect of the invention. The conditions with which completely complementary nucleic acid chains hybridize are referred to as "strict hybridization conditions" or "sequence-specific hybridization conditions" or "stringent conditions". Nevertheless, substantially complementary stable double strands of nucleic acids can be obtained under less strict or less stringent hybridization conditions, in which case, the tolerated degree of mismatch can be adjusted by means of suitable adjustment of the hybridization conditions. The person skilled in the art can empirically determine the stability of a duplex taking a number of variables into account, such as probe base pair length and concentration, ionic strength and mismatched base pair incidence, following the guidelines of the state of the art (see, for example, Sambrook et al., Molecular Cloning: A Laboratory Manual, Cold Spring Harbor Laboratory, Cold Spring Harbor, N.Y. (1989); and Wetmur, Critical Reviews in Biochem. and Mol. Biol. 26 (3/4):227-259 (1991)). The ionic strength of the hybridization conditions is related to the salt concentrations used in the hybridization solution. Adjusting the concentration of salt in the solution will have a direct effect on the stringent conditions of the hybridization. Examples of salts used in hybridization solutions are, without limitation, sodium chloride, saline sodium citrate and ammonium acetate. In a particular embodiment of the method of the invention the salinity of the sample during step (i) is equivalent to the salinity of a NaCl solution having a concentration of 0.3 M to 3 M, preferably 0.6 M to 1.2 M, more preferably 0.6 M.

In particular embodiments of the method of the invention, specific hybridization is carried out under highly restrictive conditions or under moderately restrictive conditions. Suitable conditions for said hybridization to occur are illustratively shown in Example subsection Materials and Methods - Colorimetric detections experiments of the application. As an example, and without limitation, hybridization between the target nucleic acid molecule and the second region of the oligonucleotide forming the nanoparticle of the invention takes place when 100 $\mu$L of phosphate buffer solution (PBS) 1X , 5 $\mu$L of NaCl 5M, 5 $\mu$L of the testing sample at the required concentration and 50 $\mu$L of functionalized AuNP are mixed.

As a result of said hybridization between the second region of the oligonucleotide forming the nanoparticle of the invention and a target sequence, a conformational change occurs in said oligonucleotide because the hybrid that is formed between said second region of the oligonucleotide and the target molecule is thermodynamically more stable than the nanoparticle, wherein said second region of the oligonucleotide does not hybridize with said target sequence. Thermodynamic stability can be determined by means of parameters known by the person skilled in the art, such as Gibbs free energy. Said conformational change refers to the fact that the oligonucleotide forming the nanoparticle of the invention switches from "conformation I" to "conformation II". As it is used herein, the term "conformation I of the oligonucleotide forming the nanoparticle of the invention" refers to a conformation wherein the oligonucleotide has a hairpin-type structure due to hybridization between the first and third regions of said oligonucleotide. In this conformation, the hydrophobic molecule bound to one end of the oligonucleotide is not exposed to the solvent. Said conformation I is the native conformation of the oligonucleotide forming the nanoparticle of the invention. As it is used herein, the term "conformation II of the oligonucleotide forming the nanoparticle of the invention" refers to the conformation adopted by said oligonucleotide when the second region of the oligonucleotide forming said nanoparticle hybridizes with a target sequence. As a result of said hybridization, the Watson-Crick bonds formed between the complementary nucleotides of the first and third regions of said oligonucleotide break. In this conformation II, the hydrophobic group bound to the oligonucleotide forming the nanoparticle of the invention is exposed to the solvent. The term "exposed to the solvent" refers to the fact that said group contacts with the solvent.

In a particular embodiment, said first step of the method for detecting a SARS-CoV-2 genetic material in a sample comprises contacting metal nanoparticles of the invention with a sample containing the nucleic acid to be detected, wherein the metal forming said nanoparticles is selected from of the group consisting of gold, silver, copper, aluminum, platinum, iron, cobalt, palladium and combinations thereof. In a more particular embodiment, the method for detecting a SARS-CoV-2 genetic material in a sample comprises contacting gold nanoparticles with said sample containing the nucleic acid to be detected.

As described in the context of the nanoparticles of the invention, the oligonucleotide forming the nanoparticle of the invention is modified with a hydrophobic molecule at one end and with a suitable group for the binding of the oligonucleotide to said metal nanoparticle at the end opposite to where the hydrophobic molecule is located. In a particular embodiment, said suitable group for the binding of the oligonucleotide to said nanoparticle is selected from a functional group containing a sulfur atom, a nitrogen atom or a phosphorus atom and a polyadenine sequence.

In another particular embodiment, the first step of the method for detecting SARS-CoV-2 genetic material in a test sample comprises contacting a nanoparticle according to the present invention with said sample.

In a particular and preferred embodiment, step (i) is preceded by a step of reverse transcription and amplification of the SARS-CoV-2 genetic material. This step allows for the amplification/enrichment of the genetic material before carrying out the first step of the present method of the invention. This is recommendable, although not necessary, if the amount of the genetic material to be detected is small (i.e. about picograms) in order to obtain a higher number of copies of said genetic material prior to contacting with the nanoparticle. The term "reverse transcription" as used herein refers to the process of creating a DNA molecule from RNA, sometimes called complementary DNA (cDNA). Said process is done using an enzyme called reverse transcriptase obtained from retroviruses. Methods and kits to perform said reaction are widely available for the skilled person in the art and one such method is exemplified in Examples subsection Materials and Methods - Modified RT-PCR detection experiments and Example 7 of the present description. Methods for amplifying nucleic acid molecules are known in the art, and include, but are not limited to, polymerase chain reaction (PCR), ligase chain reaction (LCR), Q-replicase amplification, degenerate oligonucleotide primed-polymerase chain reaction (DOP-PCR), T7/primase-dependent amplification, self-sustained sequence replication (3SR) and isothermal.

The term "amplified nucleic acid" in the context of the present method refers to an amplicon or amplification product such as nucleic acid that is amplified by a PCR reaction or any other method mentioned above. The amplified genetic material may be of any size depending on the region of the nucleic acid to be detected which is amplified, such as for example, between about 50 and about 100 bases pairs (bp), between about 100 bp and about 200 bp, or between 200 bp and about 500 bp, or between about 500 bp and about 1000 bp or between about 1000 bp and about 2000 bp. In a particular embodiment of the method of the invention the amplification of the genetic material is carried out using primers that produce an amplicon of about 70 bp. The term "primer" as used herein refers to a short nucleic acid sequence that provides a starting point for DNA synthesis. Normally two types of primers are used, a "forward primer" which anneals with the antisense DNA strand and initiates the synthesis of positive strand of the gene into 5' to 3' direction and a "reverse primer" which anneals with the sense strand and initiates the synthesis of the complementary strand of the coding strand, which is negative strand of the gene into 5' to 3' direction. According to the present invention, the amplified genetic material comprises the region which specifically hybridizes with the second region of the oligonucleotide forming the nanoparticle of the invention. In a preferred embodiment, the amplified genetic material comprises the entire nucleotide sequence of the genetic material to be detected. As the person skilled in the art will understand, the amplification step, preferably the PCR amplification step is carried out under suitable conditions of temperature, pH, an adequate concentration of the nucleic acid to be amplified, adequate concentrations of polymerase with suitable reactive adequate for the amplification such dNTPs or NTPs and during suitable time. If necessary, organic or inorganic chemical compounds, such as metal ions of Mg, Mn, Ca, Fe, Cu or Ni and/or cofactors, such as proteins, biotin or nicotinamide adenine dinucleotide, necessary to carry out the amplification should be added at appropriate concentration. The person skilled in the art will understand that the appropriate temperature for carry out the amplification depends on the polymerase enzyme which is used to carry out the amplification. In a preferred embodiment, the temperature for amplification is about between 20ªC and 60°C, or about 30°C or 50°C. In a still more preferred embodiment the temperature is about 45°C. Suitable time for carry out the amplification varies with temperature but is typically between 15 min and 1 day. In a preferred embodiment the amplification time is about 30 min or about 1 hour or about 2 hours or about 3 hours or about 4 hours or about 5 hours or about 6 hours or more.

In a particular embodiment of the method of the invention the amplification step is carried out using a forward primer and a reverse primer pair selected from the group consisting of: SEQ ID NO: 27 and SEQ ID NO: 28, SEQ ID NO: 29 and SEQ ID NO: 30 or SEQ ID NO: 31 and SEQ ID NO: 32. In a particular embodiment of the method of the invention the amplification step is carried out using a forward primer and a reverse primer and wherein the forward primer is modified so as to show an increased resistance to exonuclease treatment and wherein the sample is treated with an exonuclease after the amplification but prior to the contacting with the nanoparticles so the amplicons are converted to single stranded nucleic acids. The term "exonuclease" as used herein refers to enzymes that work by cleaving nucleotides one at a time from the end (exo) of a polynucleotide chain. Several types of exonucleases exist with different properties, such as, without limitation, Exonuclease I which breaks apart single-stranded DNA in a 3' to 5' direction, Exonucleases III which has a 3' to 5' exodeoxyribonuclease activity specific for double-stranded DNA and Exonuclease IV which catalyzes the degradation of double-stranded DNA by attacking the 5' terminal end. The expression "the forward primer is modified s as to show an increased resistance to exonuclease treatment" refers to the process of modifying the 5' end of the primer in order to render it less likely or impossible to be attacked by an exonuclease with a 5' to 3' catalyzing activity. In a particular embodiment said modification includes phosphorothioate bond connecting the first nucleotide at the 5' end. The "phosphorothioate bond" as used herein refers to the substitution of the sulfur atom for a non-bridging oxygen in the phosphate backbone of an oligo, rendering the internucleotide linkage resistant to nuclease degradation. In another particular embodiment said modification is a 2'-O-Methylation of the first nucleotide at the 5' end. As used herein, "2'-O-Methylation" refers to addition of a methyl group to the 2' hydroxyl of the ribose moiety of a nucleoside, rendering the oligonucleotide resistant to single-stranded endonucleases. In another particular embodiment said modification is a 2' fluorine modification of the first nucleotide at the 5' end. The term "2' fluorine modification" as used herein refers to a modified base which contains a fluorine at the 2' position (2'-fluoro). In another particular embodiment said modification is a locked nucleic acid of the first nucleotide at the 5' end. The term "locked nucleic acid" or "LNA" as used herein refers to the modification of the nucleotide wherein an extra bridge connecting the 2' oxygen and 4' carbon, locking the sugar in a 3'-endo conformation, leading to resistance to enzymatic degradation. In another particular embodiment said modification is selected from a group consisting of a phosphorothioate bond connecting the first nucleotide at the 5' end, a 2'-O-Methylation of the first nucleotide at the 5' end, a 2' fluorine modification of the first nucleotide at the 5' end, a locked nucleic acid of the first nucleotide at the 5' end.

After this amplification step, the step (i) of the method described in this aspect of the invention is carried out as detailed above.

The method of the invention also contemplates the detection of different regions of the genetic material of SARS-CoV-2 within a single detection step. Therefore, in another particular embodiment of the method of the invention, in step (i) at least two types of nanoparticles are used, wherein each type of nanoparticle is specific for a different region of the SARS-CoV-2 genetic material. In a preferred embodiment the at least two types of nanoparticles include a first type of nanoparticles in which the second region of the oligonucleotide is at least partially complementary to a region of the SARS-CoV-2 genome selected from the group consisting of the E gene, the region of the ORF1ab gene which encodes for the R protein, the region of the S gene which encodes the region of the S protein containing the S1/S2 cleavage site and the region of the S gene which encodes the region of the S protein that contains the D residue at position 614, and a second type of nanoparticles in which the second region of the oligonucleotide is at least partially complementary to a region of the SARS-CoV-2 genome selected from the group consisting of the E gene, the region of the ORF1ab gene which encodes for the R protein, the region of the S gene which encodes the region of the S protein containing the S1/S2 cleavage site and the region of the S gene which encodes the region of the S protein that contains the D residue at position 614, wherein the second region of the second type of nanoparticles is different from the first second region of the first type of nanoparticles.

The second step of the method of the invention consists in the detection of the aggregation of the nanoparticles with respect to a control sample, which indicates that the test sample contains SARS-CoV-2 genetic material. Metal nanoparticles, such as gold nanoparticles, have distinct optical and physical properties, which are dependent on their size (diameter), shape, surface structure and agglomeration state. One such distinct feature is their capacity to absorb and scatter light with extraordinary efficiency. Their strong interaction with light occurs because the conduction electrons on the metal surface undergo a collective oscillation when they are excited by light at specific wavelengths. This oscillation is known as a surface plasmon resonance (SPR), as previously explained, and it causes the absorption and scattering intensities of metal nanoparticles to be much higher than identically sized non-plasmonic nanoparticles. SPR of nanoparticles which are dispersed and stable in a solution result in a strong absorbance peak in the visible region (500-600 nm) which can be measured by UV-Visible spectroscopy and presents itself with a visible color of reddish. Once said nanoparticles are destabilized by an event such as ligand binding, nanoparticles proximity increases which may lead to their aggregation.

The optical properties of metal nanoparticles change when particles aggregate and the conduction electrons near each particle surface become delocalized and are shared amongst neighboring particles. When this occurs, the SPR shifts to lower energies, causing the absorption and scattering peaks to red-shift to longer wavelengths. Such red-shift of the UV-Vis spectrum is easily detectable by spectroscopy and may also be detectable by a change in color from reddish to blue/purple tones. In a particular embodiment of the method of the invention the detection of the aggregation of the nanoparticles is carried out by spectroscopy or naked-eye inspection, preferably spectroscopy. In a more particular embodiment the spectroscopy is UV-Vis Spectroscopy. The terms "Ultraviolet-visible spectroscopy" and/or "UV-Vis" refers to absorption spectroscopy or reflectance spectroscopy in part of the ultraviolet and the full, adjacent visible regions of the electromagnetic spectrum. This means it uses light in the visible and adjacent ranges.

The instrument used in ultraviolet-visible spectroscopy is called a UV/Vis spectrophotometer. It measures the intensity of light after passing through a sample, and compares it to the intensity of light before it passes through the sample. The UV-visible spectrophotometer can also be configured to measure reflectance. In this case, the spectrophotometer measures the intensity of light reflected from a sample, and compares it to the intensity of light reflected from a reference material (such as a white tile). UV-visible spectrophotometers use light sources to illuminate the sample which vary in the range of light wavelengths such as, and without limitation, tungsten filament from 300-2500 nm and xenon arc lamp which is continuous over 160-2,000 nm, and different detectors to detect the wavelengths returning from the sample. In addition, UV-visible spectrophotometers normally function with a scanning monochromator which moves the diffraction grating to "step-through" each wavelength so that the intensity is measured as a function of wavelength. Therefore, the absorbance spectrum of metal nanoparticles can be measured by scanning their absorbance at different wavelengths ranges, preferably from about 350-800 nm, more preferably from about 450-700 nm. As such, in a preferred embodiment of the method of the invention, the detection of aggregation of the nanoparticles in step (ii) is made by detecting a change in the spectrum peak absorbance of the test sample with respect to the control sample, wherein said change is a shift of the peak absorbance towards less energetic wavelengths (i.e., a red shift) which indicates that the test sample contains SARS-CoV-2 genetic material. As it is used herein, the term "shift of the peak absorbance towards less energetic wavelengths" refers to an at least 0.01-fold, 0.05-fold, 0.075-fold, 0.1-fold, 0.5-fold, 1-fold, 1.5-fold, 2-fold, 5-fold, 10-fold, 15-fold, 20-fold, 30-fold, 40-fold, 50-fold, 60-fold, 70-fold, 80-fold, 90-fold, 100-fold or more shift of the peak absorbance towards less energetic wavelengths in the test sample with respect to peak absorbance of the nanoparticles determined in the control sample.

Another method to determine the aggregation of nanoparticles if the test sample contains SARS-CoV-2 genetic material is by detecting the turbidity of the solution. Aggregation of nanoparticles occurs when the presence of SARS-CoV-2 genetic material leads to the unfolding of the hairpin formed by the oligonucleotide of the nanoparticle, leading to the destabilization of the nanoparticles and consequent precipitation. Said precipitation leads to a clearing of the solution and a decrease of the turbidity, due to the reduction of the scattering of the light by nanoparticles suspended in the solution. Turbidity can be measured by turbidity probes which send a beam of light to the sample and detect the light scattered by a detector normally placed at a 90 degree angle of the light source. Therefore, in a particular embodiment of the method of the invention, the detection of aggregation of the nanoparticles in step (ii) is made by detecting a reduction of the turbidity of the test sample with respect to the control sample, wherein the reduction of turbidity indicated the presence of SRAS-CoV-2 genetic material in the test sample. As it is used herein, the term "reduction of turbidity" refers to an at least 0.01-fold, 0.05-fold, 0.075-fold, 0.1-fold, 0.5-fold, 1-fold, 1.5-fold, 2-fold, 5-fold, 10-fold, 15-fold, 20-fold, 30-fold, 40-fold, 50-fold, 60-fold, 70-fold, 80-fold, 90-fold, 100-fold or more reduction of turbidity in the test sample with respect to the turbidity of the nanoparticles determined in the control sample.

The detection of the aggregation of the nanoparticles in step (ii) of the method of the invention can be carried out after a time interval that is considerable suitable for the detection of the genetic material of SARS-CoV-2, that is, a time interval which is considered ideal for the hybridization of the second region of the oligonucleotide present in the nanoparticle of step (i) of the method of the invention to hybridize with the target region of the SARS-CoV-2 genome and which will lead to a detectable change in the aggregation state of the nanoparticles. In a particular embodiment of the method of the invention the detection of the aggregation of the nanoparticles of step (ii) is done after a time interval of at least 1 min, at least 5 min, at least 10 min, at least 15 min, at least 20 min, at least 25 min, at least 30 min, preferably at least 1 h, at least 2 h, at least 3 h, at least 4 h, at least 5 h, at least 6 h, at least 7 h, at least 8 h, at least 9 h, at least 10 h. In a particular embodiment of the method of the invention the detection of the aggregation of the nanoparticles of step (ii) is done after a time interval of at least at least 12 h, at least 24 h, at least 36 h, at least 48 h, at least 60 h, at least 72 h.

The term "control sample" or "negative control" as used herein refers to a sample which goes through the same process and treatments in the same conditions as the test sample but does not contain SARS-CoV-2 genetic material and therefore no appreciable change in the physical and optical properties of the nanoparticles of the invention can be detected. The control sample may contain scramble genetic material target which does not hybridize with the second region of the oligonucleotide. In a particular embodiment of the method of the invention the control sample of step (ii) has scramble DNA as a target sequence for the second region of the oligonucleotide present in the nanoparticle of step (i). In another particular embodiment the negative control contains nanoparticles of the invention wherein the nanoparticle oligonucleotide contains a second region whose nucleotide sequence is not complementary to any of the nucleotide sequences of the SRAS-CoV-2 genetic material. The term "not complementary" as described herein refers to a nucleotide sequence with less than 10% complementarity to a nucleotide sequence of the SRAS-CoV-2 genome as previously defined.

The method of the invention also allows the detection of derivatives of the target sequence due to the detection properties. Since the detection of the aggregation of nanoparticles in step (ii) is made by quantifiable methods, such as spectroscopy and turbidity measurements, the use of a positive control allows the detection of partial hybridization between the second region of the oligonucleotide and the target sequence of the SARS-CoV-2 genetic material. Therefore, in a particular embodiment of the method of the invention, step (ii) further comprises detecting the aggregation of the nanoparticles, wherein an aggregation value of the nanoparticles in between the aggregation of a control sample and the aggregation of a positive sample indicates that the test sample contains SARS-CoV-2 genetic material and that said genetic material contains alterations in the target sequence with respect to the second region of the oligonucleotide of step (i).

The expression "aggregation of the nanoparticles in between the aggregation of a control sample and the aggregation of a positive sample" as used herein refers to an aggregation of nanoparticles which is higher or increased than the aggregation quantified in the control sample and lower or decreased than the aggregation of the positive sample.

In a particular embodiment the increase of the aggregation with respect to the control sample is of at least 1%, at least 2%, at least 3%, at least 4%, at least 5%, at least 6%, at least 7%, at least 8%, at least 9%, at least 10%, preferably at least 10%, at least 20%, at least 30%, at least 40%, at least 50%, at least 60%, at least 70%, at least 80%, at least 90%, at least 91%, at least 92%, at least 93%, at least 94%, at least 95%, at least 96%, at least 97%, at least 98%, at least 99%. In another particular embodiment the aggregation of the test sample is lower with respect to the control sample by at least 1%, at least 2%, at least 3%, at least 4%, at least 5%, at least 6%, at least 7%, at least 8%, at least 9%, at least 10%, preferably at least 10%, at least 20%, at least 30%, at least 40%, at least 50%, at least 60%, at least 70%, at least 80%, at least 90%, at least 91%, at least 92%, at least 93%, at least 94%, at least 95%, at least 96%, at least 97%, at least 98%, at least 99%.

The term "positive sample" as used herein refers to a sample which contains the target sequence with 100% identity to the second region of the oligonucleotide of the nanoparticle of step (i) allowing for a total complementary hybridization between said target sequence and said second region. Said target sequence can be produced using any method of the ones previously described.

Kits of the invention

In another aspect, the invention also contemplates kits comprising the nanoparticles according to the present invention as well as kits containing suitable reagents for preparing the nanoparticles of the invention.

Thus in one aspect, the invention relates to a first kit, hereinafter "first kit of the invention", comprising:

(i) a metal nanoparticle; and
(ii) an oligonucleotide comprising a first nucleotide region, a second nucleotide region and a third nucleotide region, in that order, wherein said first and third regions are at least partially complementary such that they can give rise to a hairpin-type structure by means of hybridization, wherein said second polynucleotide region is at least partially complementary to a region of the SARS-CoV-2 genome and wherein said oligonucleotide is modified with a hydrophobic molecule at one end and with a suitable group for the binding of the oligonucleotide to said metal nanoparticle at the end opposite to where the hydrophobic molecule is located.

In the context of the present invention, "kit" is understood as a product of the different reagents for preparing the nanoparticles of the present invention, both in those cases in which there is a single type of nanoparticles and in cases in which at least two types of nanoparticles are required (for example, when the presence of a nucleic acid with a certain mutation is to be identified), in which the different reagents are packaged together to allow for transport and storage. Nevertheless, if the kits defined in the present invention do not comprise the reagents necessary for putting the methods of the invention into practice, such reagents are commercially available and can be found as part of a kit. Suitable materials for packaging the components of the kit include, without being limited to, glass, plastic (polyethylene, polypropylene, polycarbonate and the like), bottles, vials, paper, sachets and the like. Kits can additionally contain instructions for using the different components in the kit. Said instructions can be in printed format or in an electronic device capable of storing instructions such that they can be read by a person, such as electronic storage media (magnetic discs, tapes and the like), optical means (CD-ROM, DVD, USB) and the like. The media can additionally or alternatively contain Internet addresses where said instructions are provided.

The first component first kit of the invention comprises a metal nanoparticle. The term "metal nanoparticle" and its particular embodiments are defined in the context of the first aspect of the invention and are incorporated by reference in this inventive aspect.

The second component of the first kit of the invention comprises an oligonucleotide comprising a first nucleotide region, a second nucleotide region and a third nucleotide region, in that order, wherein said first and third regions are at least partially complementary such that they can give rise to a hairpin-type structure by means of hybridization, and wherein said oligonucleotide is modified with a hydrophobic molecule at one end and with a suitable group for the binding of the oligonucleotide to said metal nanoparticle at the end opposite to where the hydrophobic molecule is located. In a particular embodiment, said suitable group for the binding of the oligonucleotide to said metal nanoparticle is selected from a functional group containing a sulfur atom, a nitrogen atom or a phosphorus atom and a polyadenine sequence.

In a particular embodiment, said oligonucleotide is modified with a functional group at the end opposite the end where it is modified with said hydrophobic molecule. In another particular embodiment, said oligonucleotide is modified with a polyadenine sequence at the end opposite the end where it is modified with said hydrophobic molecule. The terms "oligonucleotide", "hairpin-type structure", "group containing a sulfur atom", "polyadenine sequence" and "hydrophobic molecule", as well as particular embodiments thereof, are defined above in the context of the second aspect of the invention.

In a particular embodiment, the first kit of the invention additionally comprises suitable reagents for carrying out functionalization between the oligonucleotide and the metal nanoparticle of said kit. In a particular embodiment, said first kit additionally comprises suitable reagents for forming a covalent bond between the sulfur atom, the nitrogen atom or the phosphorus atom of the functional group containing said sulfur atom, said nitrogen atom or said phosphorus atom of the oligonucleotide and the metal atom of said metal nanoparticle. In another particular embodiment, said first kit additionally comprises suitable reagents for adsorption of the adenine nucleotides of said polyadenine sequence in the metal atoms of the nanoparticle. In still a more particular embodiment, the first kit of the invention comprises suitable reagents for carrying out the method for obtaining a metal nanoparticle according to the invention. Illustrative, non-limiting examples of said reagents include buffered solutions such as phosphate buffered saline (PBS), saline solutions (sodium chloride, sodium phosphate, potassium chloride, potassium phosphate), etc.

In a particular embodiment, the second component of the first kit of the invention comprises an oligonucleotide comprising a sequence which is at least partially complementary to a sequence selected from a group consisting of: SEQ ID NO: 1, SEQ ID NO: 2, SEQ ID NO: 3, SEQ ID NO: 4, SEQ ID NO: 5 and SEQ ID NO: 6.

In another particular embodiment, the second component of the first kit of the invention comprises an oligonucleotide comprising the sequence according to SEQ ID NO: 7, SEQ ID NO: 8, SEQ ID NO: 9 or SEQ ID NO: 10, SEQ ID NO: 11, SEQ ID NO: 12, SEQ ID NO: 13, SEQ ID NO: 14, SEQ ID NO: 15 or SEQ ID NO: 16.

In a particular embodiment of the first kit of the invention the kit further comprises the reagents adequate for the reverse transcription of the SARS-CoV-2 genome and reagents adequate for amplifying one or more regions of interest in the SARS-CoV-2 genome. In another preferred embodiment the reagents adequate for amplifying one or more regions of interest in the SARS-CoV-2 genome comprising a pair of primers capable of specifically amplifying said one or more regions of the SARS-CoV-2 genome. In a particular embodiment the amplification step is carried out using a forward primer and a reverse primer pair selected from the group consisting of: SEQ ID NO: 27 and SEQ ID NO: 28, SEQ ID NO: 29 and SEQ ID NO: 30 or SEQ ID NO: 31 and SEQ ID NO: 32. In a further particular embodiment the forward primer within the pair of primers has been modified so as to substantially increase its resistance to exonuclease. In yet another preferred embodiment said modification is selected from a group consisting of a phosphorothioate bond connecting the first nucleotide at the 5' end, a 2'-O-Methylation of the first nucleotide at the 5' end, a 2' fluorine modification of the first nucleotide at the 5' end, a locked nucleic acid of the first nucleotide at the 5' end.

The present invention also contemplates a kit, hereinafter "second kit of the invention", comprising the nanoparticle of the invention. All embodiments explained above in the context of the nanoparticles of the invention are equally applicable to the second kit of the invention.

The present invention also contemplates a kit, hereinafter "third kit of the invention", comprising:

(i) a metal nanoparticle;
(ii) a first oligonucleotide comprising a first nucleotide region, a second nucleotide region and a third nucleotide region, in that order, wherein said first and third regions are at least partially complementary such that they can give rise to a hairpin-type structure by means of hybridization, wherein said second polynucleotide region is at least partially complementary to a region of the SARS-CoV-2 genome and wherein said oligonucleotide is modified with a hydrophobic molecule at one end and with a suitable group for the binding of the oligonucleotide to said metal nanoparticle at the end opposite to where the hydrophobic molecule is located; and
(iii) a second oligonucleotide comprising a first nucleotide region, a second nucleotide region and a third nucleotide region, in that order, wherein said first and third regions are at least partially complementary such that they can give rise to a hairpin-type structure by means of hybridization, wherein said second polynucleotide region is at least partially complementary to a region of the SARS-CoV-2 genome and wherein said oligonucleotide is modified with a hydrophobic molecule at one end and with a suitable group for the binding of the oligonucleotide to said metal nanoparticle at the end opposite to where the hydrophobic molecule is located and wherein the second region of the oligonucleotide is different from the second region of the first oligonucleotide.

In a particular embodiment, a suitable group for the binding of the oligonucleotide to said metal nanoparticle is selected from a functional group containing a sulfur atom, a functional group containing a nitrogen atom, functional group containing a phosphorus atom and a polyadenine sequence. Therefore in a particular embodiment of the invention, said first and/or second oligonucleotide is modified with a functional group containing a sulfur atom, a nitrogen atom or a phosphorus atom at the end opposite the end where it is modified with said hydrophobic molecule. In another particular embodiment, said first and/or second oligonucleotide is modified with a polyadenine sequence at the end opposite the end where it is modified with said hydrophobic molecule

In a particular embodiment, the third kit of the invention additionally comprises suitable reagents for carrying out the functionalization between said first and second oligonucleotides and the metal nanoparticle. In a particular embodiment, said third kit of the invention additionally comprises suitable reagents for forming a covalent bond between the sulfur atom, the nitrogen atom or the phosphorus atom of the functional group containing said sulfur atom, said nitrogen atom, or said phosphorus atom of the first oligonucleotide and the metal atom of said metal nanoparticle, giving rise to a first nanoparticle according to the present invention, and/or for forming a covalent bond between the sulfur atom, the nitrogen atom or the phosphorus atom of the functional group containing said sulfur atom, said nitrogen atom, or said phosphorus atom of the second oligonucleotide and the metal atom of said metal nanoparticle, giving rise to a second nanoparticle according to the present invention. In another particular embodiment, said third kit of the invention additionally comprises suitable reagents for adsorption of the adenine nucleotides of said polyadenine sequence of the first oligonucleotide on the metal atoms of said metal nanoparticle, giving rise to a first nanoparticle according to the present invention, and/or for the adsorption of the adenine nucleotides of said polyadenine sequence of the second oligonucleotide on the metal atoms of said metal nanoparticle, giving rise to a second nanoparticle according to the present invention. In still a more particular embodiment, the third kit of the invention comprises suitable reagents for carrying out the method for obtaining a metal nanoparticle according to the invention. Illustrative, non-limiting examples of said reagents are described in the context of the first and second kits of the invention and are used with the same meaning in the context of the third kit of the invention.

In preferred embodiments, the first and second oligonucleotides of the third kit of the invention hybridize specifically with a region of the SARS-CoV-2 genome selected from the group consisting of the E gene, the region of the ORF1ab gene which encodes for the R protein, the region of the S gene which encodes the region of the S protein containing the S1/S2 cleavage site, the region of the S gene which encodes the region of the S protein that contains the D residue at position 614 and regions thereof containing mutations of clinical interest. In a preferred embodiment, the first or second oligonucleotides of the third kit of the invention hybridize specifically to a region of the E gene which corresponds to the region found between positions 26,245 and 26,472 of the SARS-CoV-2 genome as shown in the GenBank database under accession number NC_045512 (version 2 of July 18th, 2020). In another particular embodiment the first or the second oligonucleotide second nucleotide region is at least partially complementary to a region of the E gene which comprises the sequence of SEQ ID NO: 1 or SEQ ID NO: 2.

In a preferred embodiment, the first or second oligonucleotides of the third kit of the invention hybridize specifically to a region of the ORF1ab gene which encodes for the R protein which corresponds to the region found between positions 13,442 and 16,236 of the SARS-CoV-2 genome as shown in the GenBank database under accession number NC_045512 (version 2 of July 18th, 2020). In another particular embodiment the first or the second oligonucleotide second nucleotide region is at least partially complementary to a region of the E gene which comprises the sequence of SEQ ID NO: 3 or SEQ ID NO: 4.

In a preferred embodiment, the first or second oligonucleotides of the third kit of the invention hybridize specifically to a region of the S gene which corresponds to the region found between positions 21,563 and 25,384 of the SARS-CoV-2 genome as shown in the GenBank database under accession number NC_045512 (version 2 of July 18th, 2020). In another particular embodiment the first or the second oligonucleotide second nucleotide region is at least partially complementary to a region of the S gene which encodes the S1/S2 cleavage site in the spike protein which comprises the sequence of SEQ ID NO: 5 or SEQ ID NO: 6. In another particular embodiment the first or the second oligonucleotide second nucleotide region is at least partially complementary to a region of the S gene which encodes the region of the S protein which contains the D residue at position 614 and is capable of specifically hybridizing to the region of the S gene which encodes the region of the S protein which contains a D614G mutation. In particular embodiment the first or the second oligonucleotide second nucleotide region is at least partially complementary to a region of the S gene which encodes the region of the S protein which contains the D residue at position 614 which comprises the sequence of SEQ ID NO: 7, SEQ ID NO: 8, SEQ ID NO: 9, SEQ ID NO: 10, SEQ ID NO: 11 or SEQ ID NO: 12.

In a particular embodiment of the third kit of the invention the first and second oligonucleotide comprise a sequence selected from the group consisting of SEQ ID NO: 13 to SEQ ID NO: 22.

In a particular embodiment of the third kit of the invention the kit further comprises the reagents adequate for the reverse transcription of the SARS-CoV-2 genome and reagents adequate for amplifying one or more regions of interest in the SARS-CoV-2 genome. In another particular embodiment the reagents adequate for amplifying one or more regions of interest in the SARS-CoV-2 genome comprising a pair of primers capable of specifically amplifying said one or more regions of the SARS-CoV-2 genome. In a particular embodiment the amplification step is carried out using a forward primer and a reverse primer pair selected from the group consisting of: SEQ ID NO: 27 and SEQ ID NO: 28, SEQ ID NO: 29 and SEQ ID NO: 30 or SEQ ID NO: 31 and SEQ ID NO: 32. In a further particular embodiment the forward primer within the pair of primers has been modified so as to substantially increase its resistance to exonuclease. In yet another preferred embodiment said modification includes a phosphorothioate bond connecting the first nucleotides at the 5' end.

In another aspect, the invention relates to a kit, hereinafter "fourth kit of the invention", comprising:

(i) a first metal nanoparticle according to the invention; and
(ii) a second metal nanoparticle according to the invention, wherein the first and third regions of the oligonucleotide have sequences identical to the first and third regions of the oligonucleotide of the first nanoparticle, and wherein the second region of the oligonucleotide of said second nanoparticle is a variant of the second region of the oligonucleotide of the second nanoparticle.

In preferred embodiments, the first and second components of the fourth kit of the invention comprise oligonucleotides wherein the second region hybridizes with a gene of SARS-CoV-2 selected from the group consisting of E, R, S and any combination thereof, in the regions thereof containing mutations of clinical interest.

In a preferred embodiment of the invention, the first component of the fourth kit of the invention comprises a metal nanoparticle according to the invention, wherein the oligonucleotide forming said nanoparticle comprises a sequence selected from the group consisting of SEQ ID NO: 13 to SEQ ID NO: 22.

In another particular embodiment the first, second, third and fourth kit of the invention, the kit further comprises the reagents and materials required for use of the control sample and of the positive sample in order to be able to detect alterations in the target sequence of the SARS-CoV-2 genetic material in regard to the second region of the oligonucleotide of the nanoparticle. Said materials refer to the nanoparticles functionalized with the oligonucleotides or non-functionalized in which case the required reagents to functionalized the nanoparticles are also provided, buffers, solutions, salts, and the like, as well as target sequences to be used as controls.

The present invention is further characterized by the following aspects:

1. A metal nanoparticle bound to an oligonucleotide, wherein said oligonucleotide comprises a first nucleotide region, a second nucleotide region and a third nucleotide region, in that order, wherein said first and third regions are at least partially complementary such that they can give rise to a hairpin-type structure by means of hybridization, wherein said second polynucleotide region is at least partially complementary to a region of the SARS-CoV-2 genome and wherein said oligonucleotide is modified with a hydrophobic molecule at the end opposite the end bound to the metal nanoparticle.

2. The nanoparticle according to aspect 1, wherein the first and third polynucleotide regions comprise each 4-8 nucleotides.

3. The nanoparticle according to aspect 2, wherein the first and third polynucleotide regions comprise each 5 nucleotides.

4. The nanoparticle according to any of aspects 1 to 3 wherein the second nucleotide region is at least partially complementary to a region of the SARS-CoV-2 genome selected from the group consisting of the E gene, the region of the ORF1ab gene which encodes for the R protein, the region of the S gene which encodes the region of the S protein containing the S1/S2 cleavage site and the region of the S gene which encodes the region of the S protein that contains the D residue at position 614.

5. The nanoparticle according to aspect 4 wherein:

- the second nucleotide region is at least partially complementary to a region of the E gene which corresponds to the region found between positions 26,245 and 26,472 of the SARS-CoV-2 genome as shown in the GenBank database under accession number NC_045512,
- the second nucleotide region is at least partially complementary to a region of the region of the ORF1ab gene which encodes for the R protein which corresponds to the region found between positions 13,442 and 16,236 of the SARS-CoV-2 genome as shown in the GenBank database under accession number NC_045512,
- the second nucleotide region is at is at least partially complementary to a region of the S gene which corresponds to the region found between positions 21,563 and 25,384 of the SARS-CoV-2 genome as shown in the GenBank database under accession number NC_045512 or
- the second nucleotide region is at least partially complementary to a region of the S gene which encodes the region of the S protein which contains the D residue at position 614 and is capable of specifically hybridizing to the region of the S gene which encodes the region of the S protein which contains a D614G mutation.

6. The nanoparticle according to aspect 4 wherein:

- The second nucleotide region is at least partially complementary to a region of the E gene which comprises the sequence of SEQ ID NO:1,
- The second nucleotide region is at least partially complementary to a region of the ORF1ab gene which encodes for the R protein which comprises the sequence of SEQ ID NO:3,
- The second nucleotide region is at least partially complementary to a region of the S gene which encodes the S1/S2 cleavage site in the spike protein which comprises the sequence of SEQ ID NO:5 or
- the second nucleotide region is at least partially complementary to a region of the S gene which encodes the region of the S protein which contains the D residue at position 614 which comprises the sequence of SEQ ID NO:7, SEQ ID NO:9 or SEQ ID NO:11.

7. The nanoparticle according to aspect 5 wherein the oligonucleotide comprises a sequence selected from the group consisting of SEQ ID NO: 13 to 22.

8. The nanoparticle according to any of aspects 1 to 6, wherein the oligonucleotide is bound to the nanoparticle through a sulfur atom, a nitrogen atom or a phosphorus atom forming part of a group containing the sulfur atom, the nitrogen atom or the phosphorus atom.

9. The nanoparticle according to any of aspects 1 to 8 wherein the oligonucleotide and the nanoparticle are connected by a spacer region.

10. The nanoparticle according to aspect 9, wherein said spacer sequence is a polythymidine sequence and/or an aliphatic carbon chain.

11. The nanoparticle according to any of aspects 1 to 10, wherein the group containing the sulfur atom, the nitrogen atom or the phosphorus atom is bound to the 5' end of the oligonucleotide, and wherein the hydrophobic molecule is bound to the 3' end of the oligonucleotide.

12. The nanoparticle according to any of aspects 1 to 11, wherein the sulfur-metal bond is formed by a reaction between the sulfur atom of the functional group containing sulfur and a metal atom of the nanoparticle.

13. The nanoparticle according to aspect 12, wherein said functional group containing sulfur is selected from the group consisting of a thiol group, a dithiolane group and an isocyanide group.

14. The nanoparticle according to any of aspects 1 to 11, wherein the nitrogen-metal bond is formed by a reaction between the nitrogen atom of the functional group containing nitrogen and a metal atom of the nanoparticle.

15. The nanoparticle according to aspect 14, wherein said functional group containing nitrogen is selected from the group consisting of an amine, an isocyanide, a pyridine group, a purine group and a pyrimidine group.

16. The nanoparticle according to any of aspects 1 to 11, wherein the phosphorus-metal bond is formed by reaction between the phosphorus atom of the functional group containing phosphorus and a metal atom of the nanoparticle.

17. The nanoparticle according to aspect 16, wherein said functional group containing phosphorus is selected from the group consisting of a phosphine or a sulfonyldiethanol group.

18. The nanoparticle according to any of aspects 1 to 17 wherein said hydrophobic molecule is a lipid or a polycyclic aromatic hydrocarbon.

19. The nanoparticle according to aspect 18, wherein said lipid is a steroid lipid or a fatty acid.

20. The nanoparticle according to aspect 19, wherein said steroid lipid is cholesterol or a cholesterol derivative.

21. The nanoparticle according to aspect 19, wherein said fatty acid is a saturated fatty acid selected from a group consisting of: caprylic acid, capric acid, lauric acid, myristic acid, palmitic acid, stearic acid, arachidic acid, behenic acid, lignoceric acid and cerotic acid.

22. The nanoparticle according to aspect 18, wherein said polycyclic aromatic hydrocarbon is a pyrene.

23. The nanoparticle according to any of aspects 1 to 22, wherein the metal forming said nanoparticle is selected from the group consisting of gold, silver, copper, aluminum, platinum, iron, cobalt, palladium and combinations thereof.

24. The nanoparticle according to aspect 23, wherein said metal is gold.

25. The nanoparticle according to any of aspects 1 to 24 wherein the diameter of the nanoparticles is from about 15 to about 38 nm.

26. The nanoparticle according to aspect 25 wherein the diameter of the nanoparticles is from about 20 to about 25 nm.

27. The nanoparticle according to any of aspects 1 to 26 wherein the nanoparticles are stabilized so that they do not substantially aggregate when the oligonucleotide is forming a hairpin structure by the annealing of the first and third regions.

28. A process for obtaining a metal nanoparticle according to any of the preceding aspects which comprises:

(i) contacting a metal nanoparticle with an oligonucleotide, wherein the oligonucleotide comprises a first, second and third region as defined in any of the preceding aspects and wherein the oligonucleotide is modified with a group which is capable of reacting with the surface of the nanoparticle and
(ii) maintaining the nanoparticles and the oligonucleotides under suitable conditions for the formation of a bond between said oligonucleotide and the metal nanoparticle.

29. A method for detecting the presence of a SARS-CoV-2 genetic material in a test sample which comprises:

(i) contacting a nanoparticle according to any of aspects 1 to 27 with said sample wherein said contacting is carried out under conditions that allow hybridizing said region of the oligonucleotide of the nanoparticle with the nucleic acid of said sample; and
(ii) detecting aggregation of the nanoparticles, wherein an increase in aggregation with respect to a control sample indicates that the test sample contains SARS-CoV-2 genetic material.

30. The method according to aspect 29, wherein said test sample is a biological sample.

31. The method according to aspect 30, wherein said biological sample is selected from the group formed by a biological fluid, a cell lysate or a tissue lysate.

32. The method according to any of aspects 29 to 31 wherein the second region of the oligonucleotide forming part of the nanoparticles used in step (i) is at least partially complementary to the region of the S gene which encodes the region which contains the S1/S2 cleavage site, wherein an increase in the aggregation of the nanoparticles is indicative that the genetic material derives from a SARS-CoV-2 strain which contains a S1/S2 cleavage site in the spike protein.

33. The method according to any of aspects 29 to 32 wherein the second region of the oligonucleotide forming part of the nanoparticles used in step (i) is capable of specifically hybridizing with a region of the SARS-CoV-2 which contains a the mutation D614G mutation, wherein an increase in the aggregation of the nanoparticles is indicative that the genetic material derives from a SARS-CoV-2 strain which contains a D614G mutation.

34. The method according to any of aspects 29 to 33, wherein step (i) is preceded by a step of reverse transcription and amplification of the SARS-CoV-2 genetic material.

35. The method according to any of aspects 23 to 27 wherein the sample is a nasopharyngeal swab and wherein the salinity of the sample during step (i) is equivalent to the salinity of a NaCl solution having a concentration of 0,3 to 3 M.

36. The method according to aspect 34 wherein the amplification of the genetic material is carried out using primers that produce amplicons of about 70 bp.

37. The method according to aspects 34 or 35, wherein said amplification step is carried out using a forward primer and a reverse primer and wherein the forward primer is modified so as to show an increased resistance to exonuclease treatment and wherein the sample is treated with an exonuclease after the amplification but prior to the contacting with the nanoparticles so the amplicons are converted to single stranded nucleic acids.

38. The method according to aspect 36 wherein said modification in the forward primer is selected from a group consisting of a phosphorothioate bond connecting the first nucleotides at the 5' end, a 2'-O-Methylation of the first nucleotide at the 5' end, a 2' fluorine modification of the first nucleotide at the 5' end, a locked nucleic acid of the first nucleotide at the 5' end.

39. The method according to any of aspects 29 to 38 wherein in step (ii) at least two types of nanoparticles are used, wherein each type of nanoparticle is specific for a different regions of the SARS-CoV-2 genetic material.

40. The method according to aspect 38 wherein the at least two types of nanoparticles include a first type of nanoparticles in which the second region of the oligonucleotide is complementary to a region of the E gene which corresponds to the region found between positions 26,245 and 26,472 of the SARS-CoV-2 genome as shown in the GenBank database under accession number NC_045512 and a second type of nanoparticles in which the second region of the oligonucleotide is at least partially complementary to region of the ORF1 ab gene which encodes for the R protein which corresponds to the region found between positions 13,442 and 16,236of the SARS-CoV-2 genome as shown in the GenBank database under accession number NC_045512.

41. The method according to any of aspects 29 to 39 wherein the detection of the aggregation of the nanoparticles is carried out by spectroscopy.

42. A kit comprising a metal nanoparticle according to any of aspects 1 to 27.

43. The kit according to aspect 41 further comprising reagents adequate for the reverse transcription of the SARS-CoV-2 genome and reagents adequate for amplifying one or more regions of interest in the SARS-CoV-2 genome.

44. The kit according to aspect 42 wherein the reagents adequate for amplifying one or more regions of interest in the SARS-CoV-2 genome comprising a pair of primers capable of specifically amplifying said one or more regions of the SARS-CoV-2 genome.

45. The kit according to aspect 43 wherein the forward primer within the pair of primers has been modified so as to substantially increase its resistance to exonuclease.

46. The kit according to aspect 44 wherein said modification includes phosphorothioate bond connecting the first nucleotides at the 5' end.

The following examples are merely illustrative and must not be considered as limiting the present invention.

## EXAMPLES

*Materials and methods*

Oligonucleotides design, synthesis and evaluation

Molecular beacons were designed to directly recognize specific probe sequences of E and R genes, the insertion region of S protein or the mutation D614G (Table 3). The mfold Web Server from the RNA Institute College of Arts and Sciences (M. Zuker, 2003, Nucleic Acids Res., vol. 31, no. 13, pp. 3406-3415) was used to predict their folding structures and the thermodynamic parameters in the absence or presence of the targets in order to choose the best candidates. Oligonucleotides were either purchased to Integrated DNA Technologies (IDT, Belgium) or synthetized in a H8 DNA/RNA synthesizer (K&A Laborgeraete). Their purity and concentration were determined by UV-vis spectra ($\lambda$= 260 nm) in a Cary 5000 Spectrophotometer (Agilent Technologies). Scramble sequences used in the diagnosis of other microorganisms associated with respiratory infections and coinfections in COVID-19 disease (influenza virus A, influenza virus B, *Mycoplasma pneumoniae* and *Legionella pneumophila*) were used to challenge the selectivity of the system (Table 3).

*Table 3: Target sequences used in this work.*

| Target | Target Sequence |
|---|---|
| E gene | DNA: ACACTAGCCATCCTTACTGCGCTTCG (SEQ ID NO: 1)<br>RNA: ACACUAGCCAUCCUUACUGCGCUUCG (SEQ ID NO: 2) |
| R protein | DNA: CAGGTGGAACCTCATCAGGAGATG (SEQ ID NO: 3)<br>RNA: CAGGUGGAACCUCAUCAGGAGAUG (SEQ ID NO: 4) |
| Spike insertion | DNA: CTAATTCTCCTCGGCGGGCACGTAGTG (SEQ ID NO: 5)<br>RNA: CUAAUUCUCCUCGGCGGGCACGUAGUG (SEQ ID NO: 6) |

(continued)

| Target | Target Sequence |
|---|---|
| D614 | DNA: TATCAGGATGTTAAC (SEQ ID NO: 7)<br>RNA: UAUCAGGAUGUUAAC (SEQ ID NO: 8) |
| G614 (short) | DNA: TATCAGGGTGTTAAC (SEQ ID NO: 9)<br>RNA: UAUCAGGGUGUUAAC (SEQ ID NO: 10) |
| G614 (long) | DNA: TTCTTTATCAGGGTGTTAACTGCAC (SEQ ID NO: 11)<br>RNA: UUCUUUAUCAGGGUGUUAACUGCAC (SEQ ID NO: 12) |
| Influenza virus A | DNA: CTTCTAACCGAGGTCGAAACGTA (SEQ ID NO: 23) |
| Influenza virus B | DNA: TGCCTACCTGCTTTCACTAACA (SEQ ID NO: 24) |
| *Mycoplasma pneumoniae* | DNA: GAATTCCATGACATGGTA (SEQ ID NO: 25) |
| *Legionella pneumophila* | DNA: GACAAGGATAAGTTGTCTTATAGC (SEQ ID NO: 26) |

Synthesis of gold nanoparticles

Gold nanoparticles (AuNPs) of different sizes (15-38 nm) were synthesized following the Turkevich method (REF 9). Briefly, a solution of hydrogen tetrachloroaurate (III) hydrate (FisherScientific) in RNAse free water was stirred and heated at 140°C under reflux until boiling. Then, a solution of sodium citrate tribasic dehydrate, dissolved in the same water, was added and the mixture was stirred for 15 minutes. After that time, the solution was allowed to cool down at room temperature and the AuNPs obtained were filtrated through a 0.3 $\mu$m frit to get rid of large aggregates, followed by a second filtration with a PES syringe filter of 0.22 $\mu$M (MIllipore). Depending on the size of the desired AuNPs, different concentrations of reagents were used with a same final volume of 110 ml. In the case of the 15 nm AuNPs, 4.15 mM of citrate and 0.864 mM of gold (ratio of 4.8) were used. For 20-21 nm AnNPs, 3.62 mM of citrate and 0.23 mM of gold (ratio of 17.7). Finally, for the 38 nm AuNPs, 0.4 mM of citrate and 0.23 mM of gold (ratio of 1.75).

Characterization of gold nanoparticles

AuNPs' size and shape were examined by transmission electron microscopy (TEM) and Atomic Force Microscopy (AFM). Gold nanoparticles were visualized using a 120 KeV JEM1400 Flash (Jeol, Japan) at the CBMSO-CSIC. Samples were prepared by placing a glow discharged carbon shadowed formvar-coated HEX 400-mesh copper grid (AGAR) over one drop of AuNPs for 2 min and drying the excess of sample. The size and sizes distributions were determined through an automated analysis of randomly selected TEM images areas with Image J software. For AFM visualization, freshly cleaved mica was pretreated with 15 $\mu$L of poly-L-Lysine (PLL) solution 0.1% (w/v) in $H_2O$ (Sigma-Aldrich) via 10 min incubation at room temperature. The surfaces were then rinsed twice with MilliQ water and dried. Once prepared, 15 $\mu$L of vortexed and diluted nanoparticles were absorbed into the substrate following a similar procedure done with PLL. Topography images were obtained in intermittent contact mode in air using a JPK Nanowizard 2 microscope and HQXSC11-D (Mikromash) cantilevers (nominal spring constant of 42 N/m and resonance frequency of 350 kHz). Their analysis was performed using WSxM program (Horcas I., et al, 2007, Rev. Sci. Instrum., 013705) and its "flooding" tool with an N between the range of 100-1000 individual particles. The average and standard deviation of the particle size was determined using a Lorentz fitting implemented in the OriginPro software (OriginLab).

AuNPs' size and concentration were determined from UV-vis spectra in a Cary 5000 Spectrophotometer (Agilent Technologies) according to a described procedure. The size of the nanoparticles is determined by the ratio of the absorbance plasmon peak to the absorbance at 450 nm and TEM images. Based on the diameter obtained, the concentration is determined by using the proper extinction coefficient at the wavelength of 450 nm. This is only true for spherical nanoparticles (below 38nm).

AuNP-based sensor preparation

Citrate-stabilized AuNPs were functionalized with different molecular beacon oligonucleotides described in Table 1 through the formation of stable thiol-Au bonds (Zhu et al., 2020, N. Engl. J. Med., 382, 8, 727-733, 2020). The amount of the molecular beacon that can be conjugated to the nanoparticles was determined using the Equation 1:

$$Mol\ conjugated\ oligonucleotide = AAuNP \times CAuNP \times NA \times D \times VAuNP \quad [Eq.\ 1]$$

where AAuNP is the surface area of the nanoparticle ($4 \cdot \pi \cdot r2$), CAuNP is the molar concentration of AuNP in solution, D is the maximum oligonucleotide density on each particle ($3.5 \times 10^{-7}$ mol/m2) and V is the volume of AuNPs in L. According to this, AuNP of 15 nm at 15 nM can be functionalized with 2.23 pmol MB/$\mu$L de AuNP. The concentration of nanoparticles of different sizes was established in agreement with the concentration of MB mentioned before. However, the amount of MB added was twice the calculated (4.46 pmol MB/$\mu$L) to ensure that AuNP are covered with the maximum number of oligonucleotides.

Before proceeding with the functionalization, the MB were treated for 1-2h at room temperature with a 100 times excess of tris(2-carboxyethyl) phosphine hydrochloride (TCEP) to remove the protecting groups of the thiol moieties. Then, they were added to AuNP solution, vortexed, and shaken for 20 minutes for 15 nm AuNP or during 1 for bigger particles (24 and 38 nm AuNP). Then, NaCl 5M was added every 30 minutes in 5 steps to get a final concentration in the solution of 0.3M. The NaCl was mixed by quick vortexing. The AuNP were incubated overnight in darkness under shaking at RT. After that, they were washed by three cycles of centrifugation and redispersed in water. To determine the loading of oligonucleotides onto the nanoparticles, the absorbance of the supernatants was quantified ($\lambda$= 260 nm) with the Synergy H4 microplate reader, and the value was extrapolated from the corresponding calibration curve.

Different modified nanoparticles were produced containing one, two or three MB in the same nanostructure. Particularly, the sensors (AuNPs+MB) containing one MB were: E, R, S Ins, D614, G614. In the case of two MB the sensors were: E+R, E+S Ins, R+S Ins. For the preparation of this multifunctional nanoparticle, 2.23 pmol of each MB per $\mu$L of AuNP were added. The sensor combining three MB was E+R+S Ins, and in this case, the 1.49 pmol of each MB were added per $\mu$L of AuNP.

Colorimetric detection experiments

For the regular detection experiments (short targets), a 160 $\mu$L solution containing (in order of addition) 100 $\mu$L of phosphate buffer solution (PBS) 1X , 5 $\mu$L of NaCl 5M, 5 $\mu$L of the testing sample at the required concentration and 50 $\mu$L of functionalized AuNP were prepared for each replicate in 1.5 mL tubes (LabCon, USA). For the detection of longer targets (RT-PCR experiments), the NaCl concentration in the mix was raised to 1.2M.

To evaluate the functionality of the sensor, qualitative (photographs) and quantitative (absorbance measurements) studies were done at different times (1 to 24 h). For the visual readout of the sensor, photographs of the tubes placed against a white background were taken. For the quantitative determination, 70 $\mu$L of the mix were removed slowly from the upper part of the tube and the UV-Vis spectra was recorded in a Synergy H4 microplate reader (Biotek) from $\lambda$= 400 nm to $\lambda$= 650 nm in 3 nm steps. Then, the maximum absorbance peaks were used for the calculation of the % absorbance, being the control sample (without target) considered 100% of the absorbance signal. The relative absorbance (%) was calculated according to equation 2:

$$\%\ Absorbance = Sample\ absorbanceControl\ absorbance\ x\ 100\ [Eq.\ 2]$$

Sensor stability evaluation

21 nM AuNP functionalized with 4.46 pmol of MB R (stem 5)/$\mu$L AuNPs as previously described were stored under two different conditions: RT and 4°C. To evaluate the stability of the system, detection experiments after 1h in presence of 250 nM RNA short targets R (specific), E (nonspecific) or non-target (H2O) in the course of several days/months. The samples' absorbance was measured by UV-Vis spectra in a Synergy H4 microplate reader (Biotek) from $\lambda$= 400 nm to $\lambda$= 650 nm in 3 nm steps. The size of AuNP was evaluated using DLS measurements in a Zetasizer Nano-ZS device (Malvern Instruments) at different times (1 day, 3 days, 7 days, 14 days and 30 days).

Modified RT-PCR detection experiments

Total RNA extracted from infected cell cultures or patient samples were amplified by RT-PCR using the SuperScript™ III One-Step RT-PCR System with Platinum™ Taq DNA Polymerase (Invitrogen). Briefly, several individual reactions of 50 $\mu$L each were prepared according to the manufacturer's instructions with the following modifications: 20 ng of total RNA as template, 1 $\mu$M forward primer (the first 6 nucleotides at the 5' end contain phosphorothioate bond modifications), 1.2 $\mu$M reverse primer (Table 4), and 2 mM MgSO4. The cycling parameters were adjusted to the specific amplified region and are summarized in Table 5. Amplified RT-PCR products for the same target region were pooled together and loaded in a 3% agarose gel to check the success of the reaction. dsDNA products were treated with 0.4 U of T7 exonuclease (New England Biolabs; Cat. N° M0263L) per $\mu$L of RT-PCR reaction during 45 min at 37°C followed by 10 min at 80°C to digest the unprotected (non-phosphorothioated) strand. Finally, ssDNA was purified employing Monarch PCR & Cleanup Kit (New England Biolabs; Cat. N° #T1030). The purified ssDNA product was resuspended in PCR-grade water and used as target in detection experiments as described previously.

*Table 4: RT-PCR primers and cycling conditions. (\*) phosphorothioate modification.*

| Target | Primer sequence (5'-3') | Temperature °C | Time | Cycles |
|---|---|---|---|---|
| **R 74 bp fragment** | Fwd: G*T*G*T*G*G*CGGTTCACTATATGTT (SEQ ID NO: 27) Rev:GCACTATTAGCATAAGCAGTTGTG (SEQ ID NO: 28) | 55 | 1 min | 1 |
| | | 94 | 2 min | 1 |
| | | 94 | 15 s | 32 |
| | | 56.5 | 30 s | |
| | | 68 | 10 s | |
| | | 68 | 5 min | 1 |
| | | 4 | ∞ | 1 |
| **E 74 bp fragment** | Fwd: G*C*T*T*T*C*GTGGTATTCTTGCTAG(SEQ ID NO: 29) Rev: ATATTGCAGCAGTACGCACACA (SEQ ID NO: 30) | 55 | 1 min | 1 |
| | | 94 | 2 min | 1 |
| | | 94 | 15 s | 32 |
| | | 58 | 30 s | |
| | | 68 | 10 s | |
| | | 68 | 5 min | 1 |
| | | 4 | ∞ | 1 |
| **S 72 bp fragment** | Fwd: C*A*A*A*T*A*CTTCTAACCAGGTTGC ID NO: (SEQ 31) Rev: CATGAATAGCAACAGGGACTTC (SEQ ID NO: 32) | 55 | 1 min | 1 |
| | | 94 | 2 min | 1 |
| | | 94 | 15 s | 32 |
| | | 54 | 30 s | |
| | | 68 | 10 s | |
| | | 68 | 5 min | 1 |
| | | 4 | ∞ | 1 |

## *Results*

Example 1: The foundations for a Gold Nanoparticle-based sensor for COVID-19 detection

The biosensor based on functionalized gold nanoparticles for SARS-CoV-2 RNA detection was designed, synthesized, characterized and systematically evaluated in terms of stability, specificity, sensitivity and functionality in detection experiments using synthetic DNA and RNA molecules, total RNA obtained from infected cell cultures, and patients' samples.

The oligonucleotides employed contain two different domains. The central one is complementary to the selected target regions of interest in the SARS-CoV-2 genome. This domain is flanked by a few nucleotides (4-6) that stabilize the formation of a hairpin structure. (Table 2). According to the theoretical results obtained by mfold Web Server, our oligonucleotides are stable in the working conditions of the detection experiments and will selectively recognize the target sequences. These oligonucleotides contain a sulfur and a cholesterol derivative at each end. The first modification is required to conjugate the oligonucleotides to the nanoparticles, whereas the hydrophobic molecule is needed to modulate the aggregation of the nanostructures. On the other hand, we obtained AuNPs of different diameters (from 15 to 38 nm) synthesized following the Turkevich method with modifications. Their characterization by UV-spectra, TEM and AFM confirmed that we obtained colloidal dispersions of spherical gold nanoparticles of the desired sizes by adjusting the HAuCl4: citrate ratio (Figure 1). The functionalization of AuNPs with the designed molecular beacons led to stable colloidal dispersions of nanoparticles. The final loading of the MB was determined for each AuNP size.

Example 2: Optimization and stability of Gold Nanoparticle Sensors

The performance of the MB in terms of time response and specificity depends not only on the sequence design, but on its thermodynamic behavior. One parameter that determines the stability and kinetics of the molecule is the beacons' stem length. To test that, we prepared different versions of the MB using the same loop but varying the nucleotide length at the stem (4, 5 or 6 nt). The functionalization of 21 nm AuNPs with the different stem versions of the MBs E and R, alone or in combination, revealed that all sensors could recognize the DNA target sequence selectively (Figure 2). In terms of sensitivity, all the MB successfully detect the DNA target at 50 nM during the first hour. The stem of 5 nucleotides led to the fastest sensors detecting the targets [50nM target induces a decrease in the absorbance in 2 h of 65.9% (E), 66.8% (R) and 40.1% (ER)] (Figure 2). The 6 nucleotide stem time-response is similar but less efficient when a single target is detected, and worst for the ER combination [33.5% (E), 44.5% (R) and 2.5% (ER) in the same conditions]. Surprisingly, the shortest stem (4 nucleotides) led to the slowest detection sensor [-5.1% (E), 40.4% (R) and 0.1% (ER) in the same conditions], although the contrary was expected based on previous data on theoretical and experimental hybridization assays. Thus, based on these results, the stem with 5 nucleotides was selected for the following experiments.

Next, we analyzed the influence of AuNP's size in the detection of the DNA target sequences of R and E. We observed that the sensors based on the biggest nanoparticle (38 nm) were less sensitive and more unstable than the sensors prepared with AuNPs of 21 nm. On the contrary, 15 nm AuNP were much more stable, but also required longer times for the detection. To confirm these results, several batches of AuNPs between 20 and 25 nm were prepared and tested, confirming that their stability and sensitivity were optimum for our purposes. Therefore, AuNP of this range of size were chosen for the detection of these sequences instead of smaller AuNP (slow detection) and the bigger AuNP (unstable and less sensitive at low target concentrations).

Then, we confirmed that our system could also be used for the detection of RNA sequences. (Table 2). Particularly, when ssRNA sequences were used, similar results were obtained in terms of sensitivity, selectivity and detection speed (Figure 3).

Example 3: Selectivity of the system

COVID-19 patients are exposed to the potential risk of co-infection with other respiratory viruses, bacteria or fungi, increasing the difficulty of diagnosis, treatment and prognosis. From a detection point of view, the presence of those microorganisms concomitant to SARS-CoV-2 in the samples could affect or even impair the specific detection of the target virus. Thus, we evaluated if our systems present cross-reactivity with other genomic sequences from some of the most pathogens that might be present in COVID-19 patients. Particularly, representative sequences of *Influenza virus* A, *Influenza virus* B, *Mycoplasma pneumoniae* and *Legionella pneumophila,* were added to our sensors in the presence of the SARS-CoV-2 R and/or E target sequences. (Figure 4). Only when the specific SARS target was present in the mixture, we observed the expected AuNP aggregation, confirming the high selectivity of the biosensors.

Example 4: Evaluation of the sensitivity of the system

The limit of detection was assessed using RNA sequences at different concentrations (1 nM to 100 nM) (Figure 5). The system can detect the target sequences at low nanomolar, particularly the E sequence at 10 nM after 2 h, and the R one at 20 nM after 4h. Remarkably, when the system combined two sequences related to SARS-CoV-2 (E+R) the sensitivity was better (500 pM of each target, after 6 h), suggesting a cooperative effect in the functionality of the sensor. This can be exploited for the future design of sensors to improve their sensitivity and for the detection of multiple sequences.

Finally, we studied the effect of the temperature during the storage for the R sensor (5nt stem) to achieve a specific and sensitive detection of the R (RNA) target at 250nM after 1 h. Absorbance measurements using the same sensor preparations but stored at different temperatures (RT or 4°C) were done. The results showed that the functionality of the system was not affected by the storage time at both temperatures during the first 15 days, being advisable its conservation at 4°C for longer periods.

Example 5: Detection of insertions and punctual mutations in DNA target sequences coding for S protein

SARS-CoV-2 showed a high nucleotide sequence homology with other SARS-like bat coronaviruses (bat-SL-CoVZC45 and bat-SL-CoVZXC21, 88% homology) and with SARS-CoV (79.5% homology). Community transmission of the virus, as well as anti-viral treatments, can provoke novel genetic mutations with unpredictable outcomes in the clinics and the management of the infection. One of the virus genomic loci accumulating such changes is the spike gene region. The spike protein is located on the surface of the virus particle and is of great interest from a clinical, epidemiological and immunological point of view. This is because the spike protein is the ligand interacting with the cellular receptor ACE2, promoting the entry in the cells and the virus spreading through the body. This dissemination is presumably enhanced in SARS-CoV-2, compared to other related virus, due to a 12 nucleotide insertion in the spike coding sequence accounting for a Furin-like cleavage site between the S1/S2 subunits. The processing (priming) of the protein by Furin, a protease present in almost all human cell types during the biogenesis of the new virions, will pre-activate the viral progeny, boosting its transmissibility and virulence. Also, the spike protein constitutes an essential antigen for the immune system recognition and activation, and specific mutations seem to play a crucial role in the patient outcome, reinfection or vaccination efficacy. We selected as targets the insertion region (680SPRRAR↓SV687) and the mutation D614G. This last mutation is related to enhanced infectivity and stability of the virions and it is now the dominant form. Complementary MB loop sequences for this insertion (S Ins) or the D614G mutation were prepared and tested in detection experiments against DNA targets mimicking those genomic locations in the virus sequence. The results confirm the functionality of sensor S Ins in a similar way as E and R did in previous experiments, responding at 50nM DNA target concentration in 1h and even at 1nM after 24 h (Figure 6). What is more, our sensors were able to discriminate a single mismatch in the target sequence (D614G > GAT to GGT), where the G614 sensor presented greater sensitivity and velocity than that of D614 (Figure 7).

We also assessed the sensors using RNA molecules, revealing a behavior similar to the one observed with DNA derivatives. Precisely, the sensors designed to detect the insertion or the G614 mutation were able to detect their corresponding target sequences at 50 nM after 1-2 hours. On the other hand, the sensor D614 was less efficient, and the detection was achieved after 24h at 100 nM (Figure 8).

Example 6: Simultaneous detection of E, R and S Ins DNA sequences

Considering that the standard SARS-CoV-2 diagnosis implies the detection of at least two specific regions in the viral genome, we assessed the potential of this approach for the detection of multiple sequences simultaneously. Particularly, the insertion region of the spike (S) and the E and R genes (Figure 9). Remarkably, the combined detection of E or R with S led to a higher reduction of the absorbance after 2 h, 73.5% and 83.8%, respectively. The detection of the three sequences also provided an excellent reduction of absorbance over this period (77.9%). Interestingly, this multiplexed approach increases the specificity of the sensor, which is required for proper diagnosis, and also the sensitivity. However, when this approach was evaluated using RNA sequences, the sensitivity was less pronounced. In this case, the reduction in absorbance after 2 h at 50 nM of RNA for the combined detection of E+S, R+S and E+R+S, was 33.3, 26.6 and 31.3 %, respectively. Based on these results, we believe that the secondary structure formed by the RNA molecules might be preventing the efficient interaction with the oligonucleotides on the AuNPs, reducing their sensing capabilities. Thus, our system might be more suitable for the detection of DNA molecules instead of RNA. For these reasons, we explored procedures to transform the viral RNA into DNA to ease its detection, such as a modification of the standard PCR procedure described below.

Example 7: Modified PCR for the detection of the virus in patient samples using gold nanoparticles as sensors.

According to the existing data, the SARS-CoV-2 viral loads in the throat swab peaked at around 5-6 days after symptom onset, ranging from around 104 to 107 copies per mL during this time. Our sensor lowest detection limit in the best formulations (E+R) using short RNA targets (24 nt R or 26 nt E) has been 1 nM (0.5 nM each), which represents around 7.8-9.6 x $10^7$ virus copies per mL. Thus, our sensor could be used to detect the virus directly in samples with high loads, but not with samples from patients with lower virus titers. Also, the wingspan of the viral RNA and their structural complexity might decrease the sensitivity by mediating a stabilization effect, probably due to the molecular crowding effect exerted by the huge genome molecule non-interacting with the MB loop. For those reasons, we decided to couple a modified PCR-based amplification after the required transcription of the RNA into DNA. The method provides short single-stranded amplicons after exonuclease digestion of the products, which can be used in the detection experiments. In this case, we used RNA samples obtained from SARS-CoV-2 infected cells or COVID-19 nasopharyngeal patient samples with different viral loads. The PCR conditions were finely tuned to avoid false positives while keeping the direct correlation between the viral load in the samples and the amplicon performance. To maximize the aggregation effect, the primers were designed to produce short amplicons. We found that amplicons of around 70 bp were recognizable by our sensor, promoting a successfully specific hybridization followed by the desired aggregation of the nanoparticles. To improve the sensitivity and kinetics of the detection reactions, two additional modifications were implemented: (1) the increase of the NaCl concentration in the solution and (2) the extension of the loop size in the MB.

To optimize the NaCl concentration, we prepared short single-stranded amplicons of the key gene regions R (74 bp), E (74 bp) and S (72bp). The source for its production was the total RNA extracted from SARS-CoV-2 infected cells (containing the G614 mutation). Remarkably, in all cases, our system was able to detect the purified amplicons in a specific way with a sensitivity of 50 nM (E74), 100 nM (R74) or 250 nM (S72), once NaCl was adjusted to a final concentration of 1.2 M (Figure 10). In all experiments, a 72 bp GFP gene-derived fragment was used as control, which did not produce any significant aggregation. The sensor with the best performance in terms of relative absorbance changes compared with the negative control and reaction velocity was the E, followed by the S and the R. One possible explanation for this could be the different amplicon sequences and their secondary structure.

Based on these results, we decided to focus our efforts on the detection of the S gene, which is crucial for the identification of multiple variants of the virus, and our system could detect it in few hours.

Afterwards, we repeated the above determination but using purified ssDNA amplicons obtained directly from COVID-19 infected and non-infected patients (Figure 11), confirming the feasibility of the method for direct determination of SARS-CoV-2 infections. All the clinical samples used in this project were provided by the Hospital Ramon y Cajal (Madrid, Spain). The samples were inactivated using buffers containing guanidinium thiocyanate prior to RNA extraction and Ct value was determined by RT-qPCR both at the Hospital and also at IMDEA Nanociencia.

Then, once we confirmed that the approach could be used to detect the virus in patient samples, we optimized several parameters to increase its sensitivity and usability. Particularly we adjusted: (1) RT-PCR primers concentration, (2) cycling parameters, (3) NaCl concentration in the detection mix and (4) target sample volume. This optimization provides us with a method that can be used to detect the presence of the virus from patient samples accounting for different viral loads and without the need for amplicon purification. Based on these experiments, the optimal conditions were 1 $\mu$M and 1.2 $\mu$M of Forward (phosphorothioated) and Reverse primers, respectively, in a 32 cycles PCR reaction and 0.6M NaCl in the sensor mix, using 15 $\mu$l directly from the digestion reaction. In those conditions, we were capable of detecting the SARS-CoV-2 at different viral loads (Figure 12

**Claims**

1. A metal nanoparticle bound to an oligonucleotide, wherein said oligonucleotide comprises a first nucleotide region, a second nucleotide region and a third nucleotide region, in that order, wherein said first and third regions are at least partially complementary such that they can give rise to a hairpin-type structure by means of hybridization, wherein said second polynucleotide region is at least partially complementary to a region of the SARS-CoV-2 genome and wherein said oligonucleotide is modified with a hydrophobic molecule at the end opposite the end bound to the metal nanoparticle.

2. The nanoparticle according to claim 1, wherein the first and third polynucleotide regions comprise each 4-8 nucleotides.

3. The nanoparticle according to any of claims 1 or 2 wherein the second nucleotide region is at least partially complementary to a region of the SARS-CoV-2 genome selected from the group consisting of the E gene, the region of the ORF1ab gene which encodes for the R protein, the region of the S gene which encodes the region of the S protein containing the S1/S2 cleavage site and the region of the S gene which encodes the region of the S protein that contains the D residue at position 614.

4. The nanoparticle according to claim 3 wherein:

- the second nucleotide region is at least partially complementary to a region of the E gene which corresponds to the region found between positions 26,245 and 26,472 of the SARS-CoV-2 genome as shown in the GenBank database under accession number NC_045512,
- the second nucleotide region is at least partially complementary to a region of the region of the ORF1ab gene which encodes for the R protein which corresponds to the region found between positions 13,442 and 16,236 of the SARS-CoV-2 genome as shown in the GenBank database under accession number NC_045512,
- the second nucleotide region is at is at least partially complementary to a region of the S gene which corresponds to the region found between positions 21,563 and 25,384 of the SARS-CoV-2 genome as shown in the GenBank database under accession number NC_045512 or
- the second nucleotide region is at least partially complementary to a region of the S gene which encodes the region of the S protein which contains the D residue at position 614 and is capable of specifically hybridizing to the region of the S gene which encodes the region of the S protein which contains a D614G mutation.

5. The nanoparticle according to claim 4 wherein:

- The second nucleotide region is at least partially complementary to a region of the E gene which comprises the sequence of SEQ ID NO:1,
- The second nucleotide region is at least partially complementary to a region of the ORF1ab gene which encodes for the R protein which comprises the sequence of SEQ ID NO:3,
- The second nucleotide region is at least partially complementary to a region of the S gene which encodes the S1/S2 cleavage site in the spike protein which comprises the sequence of SEQ ID NO:5 or
- the second nucleotide region is at least partially complementary to a region of the S gene which encodes the region of the S protein which contains the D residue at position 614 which comprises the sequence of SEQ ID NO:7, SEQ ID NO:9 or SEQ ID NO:11.

6. The nanoparticle according to claim 5 wherein the oligonucleotide comprises a sequence selected from the group consisting of SEQ ID NO: 13 to 22.

7. A method for detecting the presence of a SARS-CoV-2 genetic material in a test sample which comprises:

(i) contacting a nanoparticle according to any of claims 1 to 6 with said sample wherein said contacting is carried out under conditions that allow hybridizing said region of the oligonucleotide of the nanoparticle with the nucleic acid of said sample; and
(ii) detecting aggregation of the nanoparticles, wherein an increase in aggregation with respect to a control sample indicates that the test sample contains SARS-CoV-2 genetic material.

8. The method according to claim 7 wherein the second region of the oligonucleotide forming part of the nanoparticles used in step (i) is at least partially complementary to the region of the S gene which encodes the region which contains the S1/S2 cleavage site, wherein an increase in the aggregation of the nanoparticles is indicative that the genetic material derives from a SARS-CoV-2 strain which contains a S1/S2 cleavage site in the spike protein.

9. The method according to claims 7 or 8 wherein the second region of the oligonucleotide forming part of the nanoparticles used in step (i) is capable of specifically hybridizing with a region of the SARS-CoV-2 which contains a the mutation D614G mutation, wherein an increase in the aggregation of the nanoparticles is indicative that the genetic material derives from a SARS-CoV-2 strain which contains a D614G mutation.

10. The method according to any of claims 7 to 9, wherein step (i) is preceded by a step of reverse transcription and amplification of the SARS-CoV-2 genetic material.

11. The method according to any of claims 7 to 10 wherein the sample is a nasopharyngeal swab and wherein the salinity of the sample during step (i) is equivalent to the salinity of a NaCl solution having a concentration of 0,3 to 3 M.

12. The method according to claims 10 or 11, wherein said amplification step is carried out using a forward primer and a reverse primer and wherein the forward primer is modified so as to show an increased resistance to exonuclease treatment and wherein the sample is treated with an exonuclease after the amplification but prior to the contacting with the nanoparticles so the amplicons are converted to single stranded nucleic acids.

13. The method according to claim 12 wherein said modification in the forward primer is selected from a group consisting of a phosphorothioate bond connecting the first nucleotides at the 5' end, a 2'-O-Methylation of the first nucleotide at the 5' end, a 2' fluorine modification of the first nucleotide at the 5' end, a locked nucleic acid of the first nucleotide at the 5' end.

14. The method according to any of claims 7 to 13 wherein in step (ii) at least two types of nanoparticles are used, wherein each type of nanoparticle is specific for a different regions of the SARS-CoV-2 genetic material.

15. The method according to claim 14 wherein the at least two types of nanoparticles include a first type of nanoparticles in which the second region of the oligonucleotide is complementary to a region of the E gene which corresponds to the region found between positions 26,245 and 26,472 of the SARS-CoV-2 genome as shown in the GenBank database under accession number NC_045512 and a second type of nanoparticles in which the second region of the oligonucleotide is at least partially complementary to region of the ORF1ab gene which encodes for the R protein which corresponds to the region found between positions 13,442 and 16,236of the SARS-CoV-2 genome as shown in the GenBank database under accession number NC_045512.

Fig. 1

Fig. 2

# E

# R

# E/R

Fig. 3

Fig. 4

Fig. 5

Fig. 6

## S Ins

## G614

## D614

Fig. 7

Fig. 8

Fig. 9

E_74nt

R_74nt

S_72nt

Fig. 10

Fig. 11

Fig. 12

Europäisches
Patentamt

European
Patent Office

Office européen
des brevets

**EUROPEAN SEARCH REPORT**

Application Number

**EP 21 38 3088**

## DOCUMENTS CONSIDERED TO BE RELEVANT

| Category | Citation of document with indication, where appropriate, of relevant passages | Relevant to claim |
|---|---|---|
| Y | WO 2015/114127 A1 (FUNDACIÓN IMDEA NANOCIENCIA [ES]; UNIV CALIFORNIA [US]) 6 August 2015 (2015-08-06) * the whole document * | 1-15 |
| Y | VICTOR M CORMAN ET AL: "Detection of 2019 novel coronavirus (2019-nCoV) by real-time RT-PCR", EUROSURVEILLANCE, vol. 25, no. 3, 23 January 2020 (2020-01-23), XP055695049, FR ISSN: 1560-7917, DOI: 10.2807/1560-7917.ES.2020.25.3.2000045 * abstract; table 1 * | 1-15 |
| Y | COUTARD B ET AL: "The spike glycoprotein of the new coronavirus 2019-nCoV contains a furin-like cleavage site absent in CoV of the same clade", ANTIVIRAL RESEARCH, ELSEVIER BV, NL, vol. 176, 10 February 2020 (2020-02-10), XP086088065, ISSN: 0166-3542, DOI: 10.1016/J.ANTIVIRAL.2020.104742 [retrieved on 2020-02-10] * abstract; figure 1 * | 1-15 |

-/--

**CLASSIFICATION OF THE APPLICATION (IPC)**

INV.
C12Q1/6816
C12Q1/70

**TECHNICAL FIELDS SEARCHED (IPC)**

C12Q

The present search report has been drawn up for all claims

| Place of search | Date of completion of the search | Examiner |
|---|---|---|
| Munich | 17 May 2022 | Bradbrook, Derek |

EPO FORM 1503 03.82 (P04C01)

page 1 of 3

EUROPEAN SEARCH REPORT

Application Number

EP 21 38 3088

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | AL-JAF SIRWAN M.A. ET AL: "Rapid, inexpensive methods for exploring SARS CoV-2 D614G mutation", META GENE, vol. 30, 18 July 2021 (2021-07-18), page 100950, XP055921620, NL ISSN: 2214-5400, DOI: 10.1016/j.mgene.2021.100950 Retrieved from the Internet: URL:https://www.sciencedirect.com/science/article/pii/S2214540021001018/pdfft?md5=b4350eabac3042e5e437e506a6acf759&pid=1-s2.0-S2214540021001018-main.pdf> * the whole document * | 1-15 | |
| A | CARVALHO JOSUÉ ET AL: "Molecular Beacon Assay Development for Severe Acute Respiratory Syndrome Coronavirus 2 Detection", SENSORS, vol. 21, no. 21, 22 October 2021 (2021-10-22), page 7015, XP055921464, DOI: 10.3390/s21217015 * the whole document * | 1-15 | TECHNICAL FIELDS SEARCHED (IPC) |
| A | ERDOGAN F ET AL: "Detection of mitochondrial single nucleotide polymorphisms using a primer elongation reaction on oligonucleotide microarrays", NUCLEIC ACIDS RESEARCH, OXFORD UNIVERSITY PRESS, GB, vol. 29, no. 7, 1 April 2001 (2001-04-01), pages E36-1, XP002287597, ISSN: 0305-1048, DOI: 10.1093/NAR/29.7.E36 * the whole document * | 12,13 | |

-/--

The present search report has been drawn up for all claims

| Place of search | Date of completion of the search | Examiner |
|---|---|---|
| Munich | 17 May 2022 | Bradbrook, Derek |

CATEGORY OF CITED DOCUMENTS

X : particularly relevant if taken alone
Y : particularly relevant if combined with another
 document of the same category
A : technological background
O : non-written disclosure
P : intermediate document

T : theory or principle underlying the invention
E : earlier patent document, but published on, or
 after the filing date
D : document cited in the application
L : document cited for other reasons
...........................................................................................
& : member of the same patent family, corresponding
 document

EPO FORM 1503 03.82 (P04C01)

page 2 of 3

Europäisches
Patentamt

European
Patent Office

Office européen
des brevets

**EUROPEAN SEARCH REPORT**

Application Number

**EP 21 38 3088**

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| T | RODRÍGUEZ DÍAZ CIRO ET AL: "Development of colorimetric sensors based on gold nanoparticles for SARS-CoV-2 RdRp, E and S genes detection", TALANTA, vol. 243, 6 March 2022 (2022-03-06), page 123393, XP055921287, NL ISSN: 0039-9140, DOI: 10.1016/j.talanta.2022.123393 ----- | | |

TECHNICAL FIELDS SEARCHED (IPC)

The present search report has been drawn up for all claims

| Place of search | Date of completion of the search | Examiner |
|---|---|---|
| Munich | 17 May 2022 | Bradbrook, Derek |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons
..............................................................................................
& : member of the same patent family, corresponding
    document

EPO FORM 1503 03.82 (P04C01)

## ANNEX TO THE EUROPEAN SEARCH REPORT
## ON EUROPEAN PATENT APPLICATION NO.

EP 21 38 3088

This annex lists the patent family members relating to the patent documents cited in the above-mentioned European search report.
The members are as contained in the European Patent Office EDP file on
The European Patent Office is in no way liable for these particulars which are merely given for the purpose of information.

17-05-2022

| Patent document cited in search report | | Publication date | Patent family member(s) | | Publication date |
|---|---|---|---|---|---|
| WO 2015114127 | A1 | 06-08-2015 | EP | 2902503 A1 | 05-08-2015 |
| | | | EP | 3099814 A1 | 07-12-2016 |
| | | | WO | 2015114127 A1 | 06-08-2015 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- Handbook of Chemistry. The Chemical Society of Japan, 168-177
- *CHEMICAL ABSTRACTS,* 57-10-3
- **YAMANA et al.** *Acids Research,* 1999, vol. 11 (1), 2387-2392
- **ZHAO et al.** *Coordination Chemistry Reviews,* 2013, vol. 257, 638-665
- Syntheses and Characterizations: 3.2 Synthesis of Metal Nanoparticles. **BRADLEY, J. S.; SCHMID, G.; TALAPIN, D. V.; SHEVCHENKO, E. V.; WELLER, H.** Nanoparticles: From Theory to Application. Wiley-VCH Verlag GmbH & Co. KGaA, 2005
- **NARANG et al.** *Meth. Enzymol.,* 1979, vol. 68, 90
- **BROWN et al.** *Meth. Enzymol.,* 1979, vol. 68, 109
- **SAMBROOK et al.** Molecular cloning: a Laboratory Manual. Cold Spring Harbor Laboratory Press, 2001, vol. 1-3
- **SAMBROOK et al.** Molecular Cloning: A Laboratory Manual. Cold Spring Harbor Laboratory, 1989
- **WETMUR.** *Critical Reviews in Biochem. and Mol. Biol,* 1991, vol. 26 (3/4), 227-259
- **M. ZUKER.** *Nucleic Acids Res.,* 2003, vol. 31 (13), 3406-3415
- **HORCAS I. et al.** *Rev. Sci. Instrum.,* 2007, 013705
- **ZHU et al.** *N. Engl. J. Med.,* 2020, vol. 382 (8), 727-733